# EUROPEAN PATENT SPECIFICATION

(11) **EP 1 979 360 B1**
(45) Date of publication and mention of the grant of the patent: **14.10.2009**
(21) Application number: 07717086.8
(22) Date of filing: 25.01.2007
(51) Int. Cl.: C07D 513/04, A61K 31/435

(54) **COMPOUNDS AND COMPOSITIONS AS PPAR MODULATORS**
VERBINDUNGEN UND ZUSAMMENSETZUNGEN ALS PPAR-MODULATOREN
COMPOSÉS ET PRÉPARATIONS UTILISÉS EN TANT QUE MODULATEURS DE PPAR

(30) Priority: 30.01.2006 US 763539 P
(43) Date of publication of application: 15.10.2008
(73) Proprietor: IRM LLC, Hamilton HM 11 (BM)
(72) Inventor: EPPLE, Robert, San Diego, California 92122 (US); RUSSO, Ross, Encinitas, California 92024 (US); AZIMIOARA, Mihai, La Jolla, California 92037 (US)
(74) Representative: Kiddle, Simon John
(86) International application number: PCT/US2007/002316
(87) International publication number: WO 2007/089667

(56) References cited:
- WO-A-20/04063155
- EICHINGER K ET AL: "ETHYNYL EPOXIDES AS SYNTHONS FOR 1,3-DIKETONES AND FURANS" JOURNAL OF CHEMICAL RESEARCH. SYNOPSES, LONDON, GB, vol. 167, no. 7, 1983, page 167, XP001014596 ISSN: 0308-2342

## Description

### Field of the Invention

The invention provides compounds, pharmaceutical compositions comprising such compounds and medical uses of using such compounds to treat or prevent diseases or disorders associated with the activity of the Peroxisome Proliferator-Activated Receptor (PPAR) families.

### Background

Peroxisome Proliferator Activated Receptors (PPARs) are members of the nuclear hormone receptor super family, which are ligand-activated transcription factors regulating gene expression. Certain PPARs are associated with a number of disease states including dyslipidemia, hyperlipidemia, hypercholesteremia, atherosclerosis, atherogenesis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, IBDs (irritable bowel disease), ulcerative colitis and Crohn's disease. Accordingly, molecules that modulate the activity of PPARs are useful as therapeutic agents in the treatment of such diseases.
WO 2004/063155 describes fused heterocyclic compounds for use as PPAR modulators.

### SUMMARY OF THE INVENTION

In one aspect, the present invention provides compounds of Formula I: 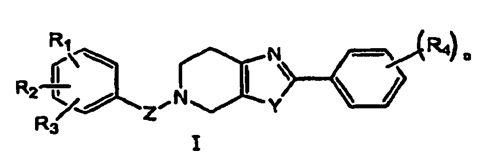 in which
n is selected from 0, 1 and 2;
R₁ is selected from-XCO₂R₁₃ and-OCR₁₁R₁₂XCO₂R₁₃; wherein X is selected from a bond and C₁₋₄alkylene; R₁₁ and R₁₂ are independently selected from hydrogen, C₁₋₄alkyl and C₁₋₄alkoxy; or R₁₁ and R₁₂ together with the carbon atom to which R₁₁ and R₁₂ are attached form C₃₋₁₂cycloalkyl; and R₁₃ is selected from hydrogen and C₁₋₆alkyl;
R₂ and R₃ are independently selected from hydrogen, halo, C₁₋₆alkyl, halo-substituted C₁₋₄alkyl, C₁₋₆alkoxy and halo-substituted-C₁₋₆alkoxy;
Z is selected from a bond and -S(O)₀₋₂-;
Y is selected from O and S;
R₄ is selected from hydrogen, halo, C₁₋₆alkyl, halo-substituted-C₁₋₆alkyl, C₁₋₆alkoxy and halo-substituted-C₁₋₆alkoxy; and the pharmaceutically acceptable salts and solvates (e.g. hydrates) of such compounds.

In a third aspect, the present invention provides the use of a compound of Formula I in the manufacture of a medicament for treating a disease in an animal in which PPAR activity activity contributes to the pathology and/or symptomology of the disease. In a fourth aspect, the present invention provides a compound of Formula I for use in a method of treatment.

### DETAILED DESCRIPTION OF THE INVENTION

### Definitions

"Alkyl" as a group and as a structural element of other groups, for example halo-substituted-alkyl and alkoxy, can be either straight-chained or branched. C₁₋₆alkoxy includes, methoxy, ethoxy, and the like. Halo-substituted alkyl includes trifluoromethyl, pentafluoroethyl, and the like.

"Aryl" means a monocyclic or fused bicyclic aromatic ring assembly containing six to ten ring carbon atoms. For example, aryl can be phenyl or naphthyl, preferably phenyl. "Arylene" means a divalent radical derived from an aryl group. "Heteroaryl" is as defined for aryl where one or more of the ring members are a heteroatom. For example heteroaryl includes pyridyl, indolyl, indazolyl, quinoxalinyl, quinolinyl, benzofuranyl, benzopyranyl, benzothiopyranyl, benzo[1,3]dioxole, imidazolyl, benzoimidazolyl, pyrimidinyl, furanyl, oxazolyl, isoxazolyl, triazolyl, tetrazolyl, pyrazolyl, thienyl, etc. "C₆₋₁₀arylC₀₋₄alkyl" means an aryl as described above connected via a alkylene grouping. For example, C₆₋₁₀arylC₀₋₄alkyl includes phenethyl, benzyl, etc.

"Cycloalkyl" means a saturated or partially unsaturated, monocyclic, fused bicyclic or bridged polycyclic ring assembly containing the number of ring atoms indicated. For example, C₃₋₁₀cycloalkyl includes cyclopropyl, cyclobutyl, cyclopentyl, cyclohexyl, etc. "Heterocycloalkyl" means cycloalkyl, as defined in this application, provided that one or more of the ring carbons indicated, are replaced by a moiety selected from -O-, -N=, -NR-, -C(O) -, -S-, -S(O) - or -S(O)₂-, wherein R is hydrogen, C₁₋₄alkyl or a nitrogen protecting group. For example, C₃₋₈heterocycloalkyl as used in this application to describe compounds of the invention includes morpholino, pyrrolidinyl, piperazinyl, piperidinyl, piperidinylone, 1,4-dioxa-8-aza-spiro[4.5]dec-8-yl, etc.

"Halogen" (or halo) preferably represents chloro or fluoro, but can also be bromo or iodo.

"Treat", "treating" and "treatment" refer to a method of alleviating or abating a disease and/or its attendant symptoms.

### Description of the Preferred Embodiments

The present invention provides compounds, compositions and medical uses for the treatment of diseases in which modulation of one or more PPARs can prevent, inhibit or ameliorate the pathology and/or symptomology of the diseases.

In one embodiment, with reference to compounds of Formula I: n is selected from 0 and 1; R₁ is selected from -XCO₂R₁₃ and-OCR₁₁R₁₂XCO₂R₁₃; wherein X is selected from a bond and C₁₋₄alkylene; R₁₁ and R₁₂ are independently selected from hydrogen, C₁₋₄alkyl and C₁₋₄alkoxy; or R₁₁ and R₁₂ together with the carbon atom to which R₁₁ and R₁₂ are attached form C₃-₁₂cycloalkyl; and R₁₃ is selected from hydrogen and C₁-₆alkyl; R₂ and R₃ are independently selected from hydrogen, halo, C₁₋₆alkyl, halo-substituted C₁₋₆alkyl, C₁₋₆alkoxy and halo-substituted-C₁₋₆alkoxy; Z is selected from a bond and -S(O)₀-2-; Y is selected from O and S; and R₄ is halo-substituted-C₁₋₆alkyl.

In another embodiment, R₁ is selected from -CH₂CO₂H, -(CH₂)CO₂H,-OC(CH₂)₂CO₂H and -OCH₂CO₂H.

In another embodiment, R₂ and R₃ are independently selected from hydrogen, methyl and methoxy; and R₄ is trifluoromethyl.

Preferred compounds of the invention are selected from: {3-[2-(4-Trifluoromethyl-phenyl)-6,7-dihydro-4H-thiazolo[5,4-c]pyridin-5-yl]-phenyl}-acetic acid; 3-{3-[2-(4-Trifluoramethyl-phenyl)-6.7-dihydro-4H-thiazolo[5.4-c]pyridin-5-yll-phenyl}-propionic acid; 3-{4-[2-(4-Trifluoromethyl-phenyl)-6,7-dihydro-4H-thiazolo[5,4-c]pyridin-5-yl]-phenyl}-propionic acid; 2-Methyl-2-{2-methyl-4-[2-(4-trifluoromethyl-phenyl)-6,7-dihydro-4H-thiazolo[5,4-c]pyridi-5-yl]-phenoxy}-propionic acid; {4-Methyl-3-[2-(4-trifluoromethyl-phenyl)-6,7-dihydro-4H-thiazolo[5,4-c]pyridine-5-sulfonyt]-phenyl}-acetic acid; {4-Methoxy-3-[2-(4-trifluoromethyl-pbenyl)-6,7-dihydro-4H-thiazolo[5.4-c]pyridine-5-sulfonyl]-phenyl}-acetic acid; {4-[2-(4-Trifluorometllyl-phenyl)-6,7-dihydro-4H-thiazolo[5,4-c]pyridine-5-sulfonyl]-phenyl}-acetic acid; {2-Methyl-4-[2-(4-trifluoromethylphenyl)-6,7-dihydro-4H-thiazolo[5,4-c]pyridine-5-sulfonyl]-phenoxy}-acetic acid; 2-Methyl-2-{2-methyl-4-[2-(4-trifluoromethyl-phenyl)-6,7-dihydro-4H-thiazolo[5,4-c]pyridine-5-sulfonyl]-phenoxy}-propionic acid; and 2-{2,5-Dimethyl-4-[2-(4-trifluoromethyl-phenyl)-6,7-dihydro-4H-thiazolo[5,4-c]pyridine-5-sulfonyl]-phenoxy}-2-methyl-propionic acid.

Further preferred compounds and intermediates of the invention are detailed in the Examples, *infra.*

### Pharmacology and Utility

Compounds of the invention modulate the activity of PPARs and, as such, are useful for treating diseases or disorders in which PPARs contributes to the pathology and/or symptomology of the disease. This invention further provides compounds of this invention for use in the preparation of medicaments for the treatment of diseases or disorders in which PPARs contributes to the pathology and/or symptomology of the disease.

Such compounds may therefore be employed for the treatment of prophylaxis, dyslipidemia, hyperlipidemia, hypercholesteremia, atherosclerosis, atherogenesis, hypertriglyceridemia, heart failure, hyper cholesteremia, myocardial infarction, vascular diseases, cardiovascular diseases, hypertension, obesity, cachexia, HIV wasting syndrome, inflammation, arthritis, cancer, Alzheimer's disease, anorexia, anorexia nervosa, bulimia, skin disorders, respiratory diseases, ophthalmic disorders, IBDs (irritable bowel disease), ulcerative colitis and Crohn's disease. Preferably for the treatment of prophylaxis, dyslipidemia, hyperlipidemia, hypercholesteremia, atherosclerosis, atherogenesis, hypeririglyceridemia, cardiovascular diseases, hypertension, obesity, inflammation, cancer, skin disorders, IBDs (irritable bowel disease), ulcerative colitis and Crohn's disease.

Compounds of the invention can also be employed to treat long term critical illness, increase muscle mass and/or muscle strength, increase lean body mass, maintain muscle strength and function in the elderly, enhance muscle endurance and muscle function, and reverse or prevent frailty in the elderly.

Further, the compounds of the present invention may be employed in mammals as hypoglycemic agents for the treatment and prevention of conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome X. Preferably type-1 and type-2 diabetes, Impaired Glucose Metabolism (IGM), Impaired Glucose Tolerance (IGT) and Impaired Fasting Glucose (IFG).

In accordance with the foregoing, the present invention also concerns: i) a compound of the invention or a pharmaceutically acceptable salt thereof for use as a medicament; and ii) the use of a compound of the invention or a pharmaceutically acceptable salt thereof for the manufacture of a medicament for presenting or treating any of the diseases or disorders described above.

### Administration and Pharmaceutical Compositions

In general, compounds of the invention will be administered in therapeutically effective amounts via any of the usual and acceptable modes known in the art, either singly or in combination with one or more therapeutic agents. A therapeutically effective amount can vary widely depending on the severity of the disease, the age and relative health of the subject, the potency of the compound used and other factors. In general, satisfactory results are indicated to be obtained systemically at daily dosages of from about 0.03 to 2.5mg/kg per body weight. An indicated daily dosage in the larger mammal, e.g. humans, is in the range from about 0.5mg to about 100mg, conveniently administered, e.g. in divided doses up to four times a day or in retard form. Suitable unit dosage forms for oral administration comprise from ca. 1 to 50mg active ingredient.

Compounds of the invention can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, oral compositions can be tablets or gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrollidone; if desired d) disintegrants, e.g., starches, agar, alginic acid or its sodium salt, or effervescent mixtures; and/or e) absorbents, colorants, flavors and sweeteners. Injectable compositions can be aqueous isotonic solutions or suspensions, and suppositories can be prepared from fatty emulsions or suspensions. The compositions can be sterilized and/or contain adjuvants, such as preserving, stabilizing, wetting or emulsifying agents, solution promoters, salts for regulating the osmotic pressure and/or buffers. In addition, they can also contain other therapeutically valuable substances. Suitable formulations for transdermal applications include an effective amount of a compound of the present invention with a carrier. A carrier can include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin. Matrix transdermal formulations can also be used. Suitable formulations for topical application, e.g., to the skin and eyes, are preferably aqueous solutions, ointments, creams or gels well-known in the art. Such can contain solubilizers, stabilizers, tonicity enhancing agents, buffers and preservatives.

This invention also concerns a pharmaceutical composition comprising as therapeutically effective amount of a compound as described herein in combination with one or more pharmaceutically acceptable carriers.

Compounds of the invention can be administered in therapeutically effective amounts in combination with one or more therapeutic agents (pharmaceutical combinations).

Thus, the present invention also relates to pharmaceutical combinations, such as a combined preparation or pharmaceutical composition (fixed combination), comprising: 1) a compound of the invention as defined above or a pharmaceutical acceptable salt thereof; and 2) at least one active ingredient selected from:
a) anti-diabetic agents such as insulin, insulin derivatives and mimetics; insulin secretagogues such as the sulfonylureas, e.g., Glipizide, glyburide and Amaryl; insulinotropic sulfonylurea receptor ligands such as meglitinides, e.g., nateglinide and repaglinide; insulin sensitizer such as protein tyrosine phosphatase-1B (PTP-1B) inhibitors such as PTP-112; GSK3 (glycogen synthase kinase-3) inhibitors such as SB-517955, SB-4195052, SB-216763, NN-57-05441 and NN-57-05445; RXR ligands such as GW-0791 and AGN-194204; sodium-dependent glucose co-transporter inhibitors such as T-1095; glycogen phosphorylase A inhibitors such as BAY R3401; biguanides such as metformin; alpha-glucosidase inhibitors such as acarbose; GLP-1 (glucagon like peptide-1), GLP-1 analogs such as Exendin-4 and GLP-1 mimetics; DPPIV (dipeptidyl peptidase IV) inhibitors such as DPP728, LAF237 (vildagliptin - Example 1 of WO 00/34241), MK-0431, saxagliptin, GSK23A; an AGE breaker, a thiazolidone derivative (glitazone) such as pioglitazone, rosiglitazone, or (R)-1-{4-[5-methyl-2-(4-tnfluoromethyl-phenyl)-oxazol-4-ylmethoxy]-benzenesulfonyl}-2,3-dihydro-1H-indole-2-carboxylic acid described in the patent application WO 03/043985, as compound 19 of Example 4, a non-glitazone type PPARγ agonist e.g. GI-262570;
b) hypolipidemic agents such as 3-hydroxy-3-methyl-glutaryl coenzyme A (HMG-CoA) reductase inhibitors, e.g., lovastatin, pitavastatin, sirnvastatin, pravastatin, cerivastatin, mevastatin, velostatin, fluvastatin, dalvastatin, atorvastatin, rosuvastatin and rivastatin; squalene synthase inhibitors; FXR (farnesoid X receptor) and LXR (liver X receptor) ligands; cholestyramine; fibrates; nicotinic acid and aspirin;
c) an anti-obesity agent or appetite regulating agent such as phentetmine, leptin, bromocriptine, dexamphetamine, amphetamine, fenfluramine, dexfenfluramine, sibutramine, orlistat, dexfenfluramine, mazindol, phenternine, phendimetrazine, diethylpropion, fluoxetine, bupropion, topiramate, diethylpropion, benzphetamine, phenylpropanolamine or ecopipam, ephedrine, pseudoephedrine or cannabinoid receptor antagonists;
d) anti-hypertensive agents, e.g., loop diuretics such as ethacrynic acid, furosemide and torsemide; diuretics such as thiazide derivatives, chlorithiazide, hydrochlorothiazide, amiloride; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors e.g. thiorphan, terteo-thiorphan, SQ29072; ECE inhibitors e.g. SLV306; ACE/NEP inhibitors such as omapatrilat, sampatrilat and fasidotril; angiotensin II antagonists such as candesartan, eprosartan, irbesartan, losartan, telmisartan and valsartan, in particular valsartan; renin inhibitors such as aliskiren, terlakiren, ditekiren, RO 66-1132, RO-66-1168;.β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; and aldosterone synthase inhibitors;
e) a HDL increasing compound;
f) Cholesterol absorption modulator such as Zetia® and KT6-971;
g) Apo-A1 analogues and mimetics;
h) thrombin inhibitors such as Ximelagatran;
i) aldosterone inhibitors such as anastrazole, fadrazole, eplerenone;
j) Inhibitors of platelet aggregation such as aspirin, clopidogrel bisulfate;
k) estrogen; testosterone; a selective estrogen receptor modulator, a selective androgen receptor modulator;
l) a chemotherapeutic agent such as aromatase inhibitors e.g. femara, anti-estrogens, topoisomerase I inhibitors, topoisomerase II inhibitors, microtubule active agents, alkylating agents, antineoplastic antimetabolites, platin compounds, compounds decreasing the protein kinase activity such as a PDGF receptor tyrosine kinase inhibitor preferably Imatinib ( {N-{5-[4-(4-methyl-piperazino-methyl)-benzoylamido]-2-methylphenyl}-4-(3-pyridyl)-2-pyrinudine-amine }) described in the European patent application EP-A-0 564 409 as example 21 or 4-Methyl-N-[3-(4-methyl-imidazol-1-yl)-5-trifluoromethyl-phenyl]-3-(4-pyridin-3-yl-pyrimidin-2-ylamino)-benzamide described in the patent application WO 04/005281 as example 92; and
m) an agent interacting with a 5-HT₃ receptor and/or an agent interacting with 5-HT₄ receptor such as tegaserod described in the US patent No. 5510353 as example 13, tegaserod hydrogen maleate, cisapnde, cilansetron;
or, in each case a pharmaceutically acceptable salt thereof; and optionally a pharmaceutically acceptable carrier.

Most preferred combination partners are tegaserod, imatinib, vildagliptin, metformin, a thiazolidone derivative (glitazone) such as pioglitazone, rosiglitazone, or (*R*)-1-{4-[5-methyl-2-(4-trifluoromethyl-phenyl)-oxazol-4-ylmethoxy]-benzenesulfonyl} -2,3-dihydro-1*H*-indole-2-carboxylic acid, a sulfonylurea receptor ligand, aliskiren, valsartan, orlistat or a statin such as pitavastatin, simvastatin, fluvastatin or pravastatin.

Preferably the pharmaceutical combinations contains a therapeutically effective amount of a compound of the invention as defined above, in a combination with a therapeutically effective amount of another therapeutic agent as described above, e.g., each at an effective therapeutic dose as reported in the art. Combination partners (1) and (2) can be administered together, one after the other or separately in one combined unit dosage form or in two separate unit dosage forms. The unit dosage form may also be a fixed combination.

The structure of the active agents identified by generic or trade names may be taken from the actual edition of the standard compendium "The Merck Index" or the Physician's Desk Reference or from databases, e.g. Patents International (e.g. IMS World Publications) or Current Drugs. The corresponding content thereof is hereby incorporated by reference. Any person skilled in the art is fully enabled to identify the active agents and, based on these references, likewise enabled to manufacture and test the pharmaceutical indications and properties in standard test models, both in vitro and in vivo.

In another preferred aspect the invention concerns a pharmaceutical composition (fixed combination) comprising a therapeutically effective amount of a compound as described herein, in combination with a therapeutically effective amount of at least one active ingredient selected from the above described group a) to m), or, in each case a pharmaceutically acceptable salt thereof.

A pharmaceutical composition or combination as described herein for the manufacture of a medicament for the treatment of for the treatment of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome-X.

Such therapeutic agents include estrogen, testosterone, a selective estrogen receptor modulator, a selective androgen receptor modulator, insulin, insulin derivatives and mimetics; insulin secretagogues such as the sulfonylureas, e.g., Glipizide and Amaryl; insulinotropic sulfonylurea receptor ligands, such as meglitinides, e.g., nateglinide and repaglinide; insulin sensitizers, such as protein tyrosine phosphatase-1B (PTP-1B) inhibitors, GSK3 (glycogen synthase kinase-3) inhibitors or RXR ligands; biguanides, such as metformin; alpha-glucosidase inhibitors, such as acarbose; GLP-1 (glucagon like peptide-1), GLP-1 analogs, such as Exendin-4, and GLP-1 mimetics; DPPIV (dipeptidyl peptidase IV) inhibitors, e.g. isoleucin-thiazolidide; DPP728 and LAF237, hypolipidemic agents, such as 3-hydroxy-3-methyl-glutaryl coenzyme A (HMG-CoA) reductase inhibitors, e.g., lovastatin, pitavastatin, simvastatin, pravastatin, cerivastatin, mevastatin, velostatin, fluvastatin, dalvastatin, atorvastatin, rosuvastatin, fluindostatin and rivastatin, squalene synthase inhibitors or FXR (liver X receptor) and LXR (farnesoid X receptor) ligands, cholestyramine, fibrates, nicotinic acid and aspirin. A compound of the present invention may be administered either simultaneously, before or after the other active ingredient, either separately by the same or different route of administration or together in the same pharmaceutical formulation.

The invention also provides for pharmaceutical combinations, e.g. a kit, comprising: a) a first agent which is a compound of the invention as disclosed herein, in free form or in pharmaceutically acceptable salt form, and b) at least one co-agent. The kit can comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time. The term "pharmaceutical combination" as used herein means a product that results from the mixing or combining of more than one active ingredient and includes both fixed and non-fixed combinations of the active ingredients. The term "fixed combination" means that the active ingredients, e.g. a compound of Formula I and a co-agent, are both administered to a patient simultaneously in the form of a single entity or dosage. The term "non-fixed combination" means that the active ingredients, e.g, a compound of Formula I and a co-agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

### Processes for Making Compounds of the Invention

Compounds of the present invention may be prepared as follows. In the reactions described, it can be necessary to protect reactive functional groups, for example hydroxy, amino, imino, thio or carboxy groups, where these are desired in the final product, to avoid their unwanted participation in the reactions. Conventional protecting groups can be used in accordance with standard practice, for example, see T.W. Greene and P. G. M. Wuts in "Protective Groups in Organic Chemistry", John Wiley and Sons, 1991.

Compounds of Formula 4 can be prepared by proceeding as in reaction scheme 1: 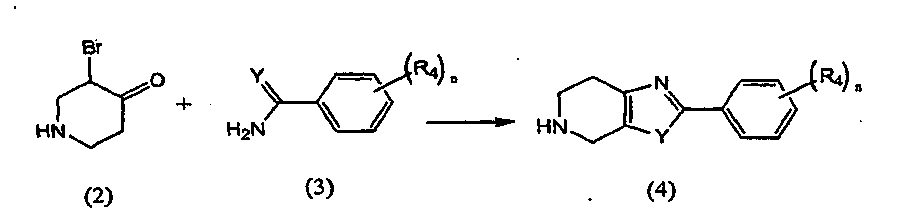 in which n, Y and R₄ are as defined for Formula I. Compounds of Formula 4 are prepared by reacting a compound of formula 2 (preferably a hydrobromide or hydrochloride salt, thereof) with a compound of formula 3 in the presence of a suitable solvent (for example, DMF, and the luke). The reaction is carried out in the temperature range of about 50 to about 125°C and takes up to about 24 hours to complete.

Compounds of Formula I, in which Z is a bond, can be prepared by proceeding as in reaction scheme 2: 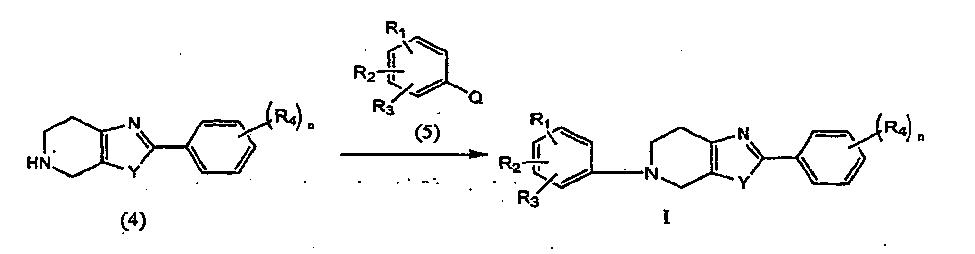 in which n, Y, R₁, R₂, R₃ and R₄ are as defined for Formula I and Q is chloro, bromo or iodo. Compounds of Formula I are prepared by reacting a compound of formula 4 with a compound of formula 5 in the presence of a suitable solvent (for example, dioxane, and the like), a suitable catalyst (for example; Pd₂(dba)₃, and the like), a suitable ligand (for example, phosphine ligands such as (tBU)₃PHBF₃, and the like), a suitable inorganic base (for example, Cesium carbonate, and the like) under a suitable protective atmosphere (for example, argon, and the like). The reaction is carried out in the temperature range of about 80 to about 150°C and takes up to about 24 hours to complete.

Compounds of Formula I, in which Z is -S(O)₀₋₂-, can be prepared by proceeding as in reaction scheme 3: 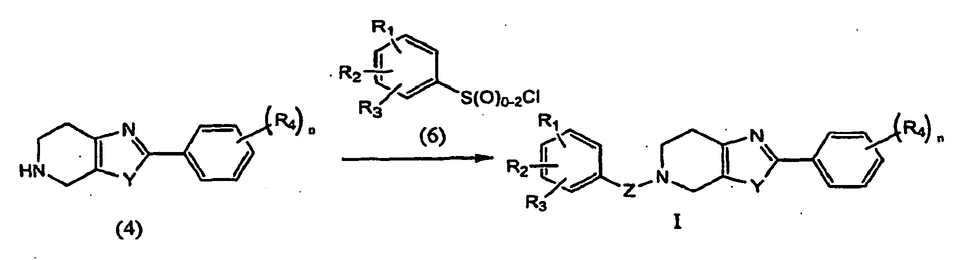 in which n, Y, R₁, R₂, R₃ and R₄ are as defined for Formula I and Q is chloro, bromo or iodo. Compounds of Formula I are prepared by reacting a compound of formula 4 with a compound of formula 6 in the presence of a suitable solvent (for example, DCM, and the like), a suitable organic base (for example, triethylamine, and the like). The reaction is carried out in the temperature range of about 0 to about 50°C and takes up to about 24 hours to complete.

Compounds of Formula I, where R₁ is selected from -XCO₂R₁₃ and - OCR₁₁R₁₂XCO₂R₁₃ (and R₁₃ is C₁₋₆alkyl), are converted to their corresponding acids (where R₁₃ is hydrogen) via a saponification reaction. The reacting proceeds in the presence of a suitable base (e.g., lithium hydroxide, or the like) and a suitable solvent mixture (e.g., THF/water, or the like) and is carried out in the temperature range of about 0°C to about 50°C, taking up to about 30 hours to complete.

Detailed reaction conditions are described in the examples, *infra*.

### Additional Processes for Making Compounds of the Invention

A compound of the invention can be prepared as a pharmaceutically acceptable acid addition salt by reacting the free base form of the compound with a pharmaceutically acceptable inorganic or organic acid. Alternatively, a pharmaceutically acceptable base addition salt of a compound of the invention can be prepared by reacting the free acid form of the compound with a pharmaceutically acceptable inorganic or organic base. Alternatively, the salt forms of the compounds of the invention can be prepared using salts of the starting materials or intermediates.

The free acid or free base forms of the compounds of the invention can be prepared from the corresponding base addition salt or acid addition salt from, respectively.
For example a compound of the invention in an acid addition salt form can be converted to the corresponding free base by treating with a suitable base (e.g., ammonium hydroxide solution, sodium hydroxide, and the like). A compound of the invention in a base addition salt form can be converted to the corresponding free acid by treating with a suitable acid (e.g., hydrochloric acid, etc.).

Compounds of the invention in unoxidized form can be prepared from N-oxides of compounds of the invention by treating with a reducing agent (e.g., sulfur, sulfur dioxide, triphenyl phosphine, lithium borohydride, sodium borohydride, phosphorus trichloride, tribromide, or the like) in a suitable inert organic solvent (e.g. acetonitrile, ethanol, aqueous dioxane, or the like) at 0 to 80°C.

Prodrug derivatives of the compounds of the invention can be prepared by methods known to those of ordinary skill in the art (e.g., for further details see Saulnier et al., (1994), Bioorganic and Medicinal Chemistry Letters, Vol. 4, p. 1985). For example, appropriate prodrugs can be prepared by reacting a non-derivatized compound of the invention with a suitable carbamylating agent (e.g., 1,1-acyloxyalkylcarbanochloridate, paranitrophenyl carbonate, or the like).

Protected derivatives of the compounds of the invention can be made by means known to those of ordinary skill in the art. A detailed description of techniques applicable to the creation of protecting groups and their removal can be found in T. W. Greene, "Protecting Groups in Organic Chemistry", 3rd edition, John Wiley and Sons, Inc., 1999.

Compounds of the present invention can be conveniently prepared, or formed during the process of the invention, as solvates (e.g., hydrates). Hydrates of compounds of the present invention can be conveniently prepared by recrystallization from an aqueous/organic solvent mixture, using organic solvents such as dioxin, tetrahydrofuran or methanol.

Compounds of the invention can be prepared as their individual stereoisomers by reacting a racemic mixture of the compound with an optically active resolving agent to form a pair of diastereoisomeric compounds, separating the diastereomers and recovering the optically pure enantiomers. While resolution of enantiomers can be carried out using covalent diastereomeric derivatives of the compounds of the invention, dissociable complexes are preferred (e.g., crystalline diastereomeric salts). Diastereomers have distinct physical properties (e.g., melting points, boiling points, solubilities, reactivity, etc.) and can be readily separated by taking advantage of these dissimilarities. The diastereomers can be separated by chromatography, or preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques, Andre Collet, Samuel H. Wilen, "Enantiomers, Racemates and Resolutions", John Wiley And Sons, Inc., 1981.

In summary, the compounds of Formula I can be made by a process, which involves:
(a) that of reaction scheme above; and
(b) optionally converting a compound of the invention into a pharmaceutically acceptable salt;
(c) optionally converting a salt form of a compound of the invention to a non-salt form;
(d) optionally converting an unoxidized form of a compound of the invention into a pharmaceutically acceptable .N-oxide;
(e) optionally converting an N-oxide form of a compound of the invention to its unoxidized form;
(f) optionally resolving an individual isomer of a compound of the invention from a mixture of isomers;
(g) optionally converting a non-derivatized compound of the invention into a pharmaceutically acceptable prodrug derivative; and
(h) optionally converting a prodrug derivative of a compound of the invention to its non-derivatized form.

Insofar as the production of the starting materials is not particularly described, the compounds are known or can be prepared analogously to methods known in the art or as disclosed in the Examples hereinafter.

One of skill in the art will appreciate that the above transformations are only representative of methods for preparation of the compounds of the present invention, and that other well known methods can similarly be used.

### Example

The present invention is further exemplified, but not limited, by the following intermediates and examples that illustrate the preparation of compounds of Formula I according to the invention.

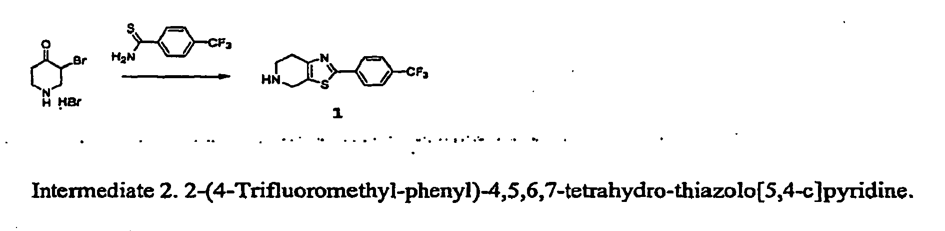

Step A: 3-Bromo-piperidin-4-one hydrobromide 1 (1.5 g, 5.79 mmol) and 4-trifluoromethyl-thiobenzamide (0.79 g, 3.96 mmol) are dissolved in DMF (2 mL) and heated to 100°C for 8 h. After cooling to room temperature the mixture is diluted with EtOAc and extracted with water. The aqueous layer containing the protonated product is basified with aqueous saturated bicarbonate and extracted with DCM twice. The organic layers are combined and concentrated to afford 2 (0.45 g, 40%) as a reddish solid: ¹H-NMR (400MHz, CDCl₃) δ = 8.01 (d, J = 8.1 Hz, 2H), 7.67 (d, J = 8.1 Hz, 2H), 4.12 (t, J = 1.7 Hz, 2H), 3.24 (t, J = 5.8 Hz, 2H), 2.91 (m, 2H). MS calcd. for C₁₃H₁₂F₃N₂S (M+H⁺) 285.1, found 285.1.

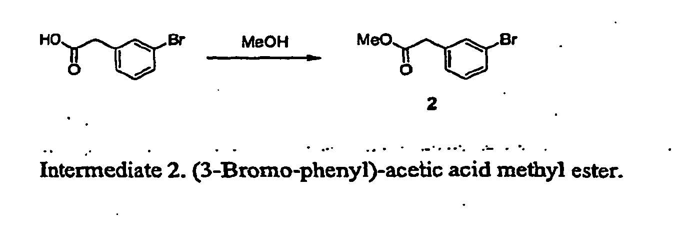

Step A: 3-Bromophenyl acetic acid (1.17 g, 5.44 mmol) is dissolved in MeOH (15 mL) containing catalytic amounts of thionyl chloride (0.2 mL). The solution is stirred at room temperature overnight. The solvent is evaporated, the remainder is dissolved in DCM and washed with water and saturated aqueous NaHCO₃. The organic layer is dried (MgSO₄), filtered and concentrated to afford the methyl ester 16 (1.20 g, 5.28 mmol, 96%) as an oil: ¹H-NMR (400MHz, CDCl₃) δ = 7.44 (s, 1H), 7.40 (ddd, J = 2.0, 2.4, 6.8 Hz, 1H), 7.20 (m, 2H), 3.70 (s, 3H), 3.59 (s, 2H). MS calcd. for C₉H₁₀BrO₂ (M+H⁺) 229.1, found 229.0.

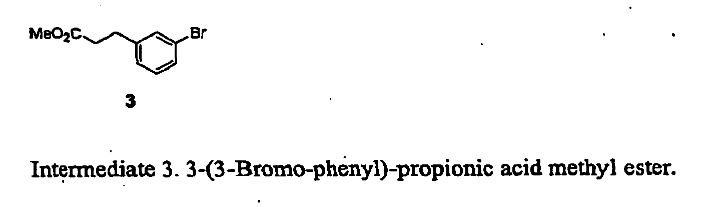

Following the procedure for Intermediate 2, except substituting 3-(3-bromo-phenyl)-propionic acid for 3-bromophenyl acetic acid, the title compound is prepared as a clear liquid: MS calcd. for C₁₀H₁₀BrO₂ (M+H⁺) 243.0, found 243.1.

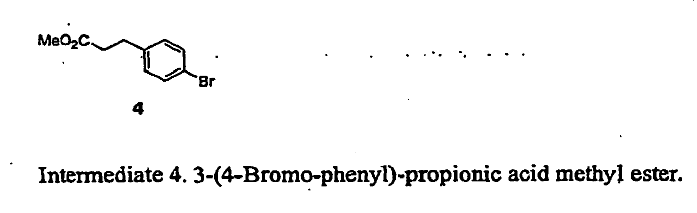

Following the procedure for Intermediate 2, except substituting 3-(4-Bromo-phenyl)-propionic acid for-3-bromophenyl acetic acid, the title compound is prepared as a clear liquid: MS calcd. for C₁₀H₁₁ BrO₂ (M+H⁺) 243.0, found 243.1.

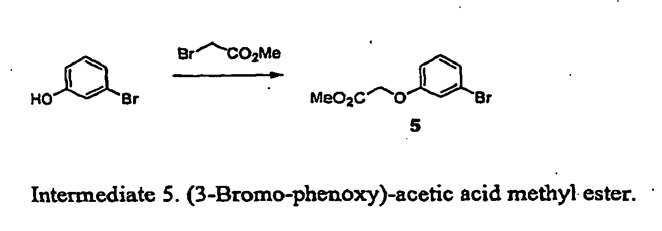

3-Bromo-phenol (1.72 g, 10 mmol) together with methyl-bromoacetate (1.01 mL, 11 mmol) are dissolved in MeCN (600 mL). K₂CO₃ (2.07 g, 15 mmol) is added and the mixture is stirred at 50°C overnight. After insoluble salts are filtered and washed with MeCN, the solvent is removed and the remainder is taken up in EtOAc and washed subsequently with water and brine. The organic layer is dried (MgSO₄), filtered and concentrated to afford 5 (2.31 g, 95%) as a colourless semi-solid: MS calcd. for C₉H₁₀BrO₃ (M+H⁺) 245.0, found 244.9.

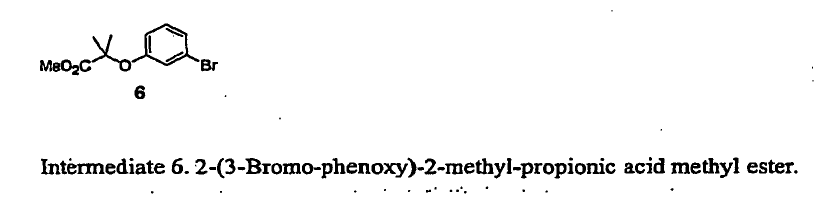

Following the procedure of Intermediate 5, except substituting α,α-dimethyl-methyl-bromoacetate for methyl-bromoacetate and heating to reflux, the title compound is prepared as a clear liquid: MS calcd. for C₁₁H₁₄BrO₃ (M+H⁺) 273.0, found 273.0.

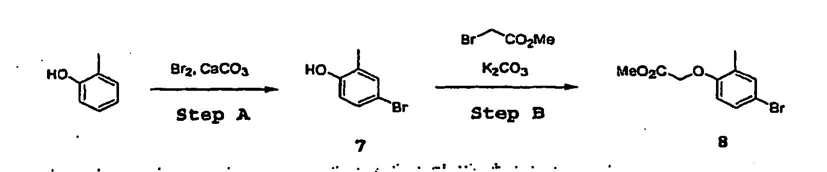

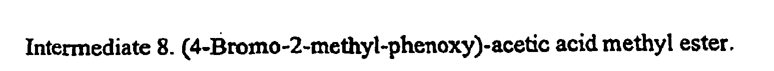

Step A: o-Cresol (10.8 g, 0.10 mol) is dissolved in DCM (100 mL). CaCO₃ (15 g, 0.15 mol) is added and the mixture is cooled to 0°C. Then bromine (5.14 mL, 0.10 mol) dissolved in DCM (100 mL) is added dropwise over a period of 30 min. Sodiun sulfite is added to quench the bromine, the mixture is warmed to room temperature and diluted with more DCM (200 mL). After the addition of MgSO₄ the mixture is filtered, the filtrate is concentrated to give 4-bromo-2-methyl-phenol 7 (17.5 g, 94%) as a white solid: ¹H-NMR (400MHz, CDCl₃) δ = 7.24 (d, J = 2.3 Hz, 1H), 7.17 (dd, J = 2.3 Hz, J = 8.5 Hz, 1H), 6.65 (d, J = 8.5 Hz, 1H), 4.69 (s, OH), 2.22 (s, 3H). MS calcd. for C₁₁H₁₄BrO₃ (M+H⁺) 187.0, found 186.9.

Step B: 4-Bromo-2-methyl-phenol 7 (3.74 g, 20 mmol) together with methyl-bromoacetate (2.03 mL, 22 mmol)-are dissolved in MeCN (200 mL). K₂CO₃ (4.15 g, 30 mmol) is added and the mixture is stirred at 50°C overnight. After insoluble salts are filtered and washed with MeCN, the solvent is removed and the remainder is taken up in EtOAc and washed subsequently with water and brine. The organic layer is dried (MgSO₄), filtered and concentrated to afford 8 (4.66 g, 90%) as a colourless oil: ¹H-NMR (400MHz, CDCl₃) δ = 7.28 (d, J = 2.4 Hz, 1H), 7.22 (dd, J = 2.4 Hz, J = 8.6 Hz, 1H), 6.56 (d, J = 8.6 Hz, 1H), 4.63 (s, 2H), 2.26 (s, 3H). MS calcd. for C₁₀H₁₂BrO₃ (M+H⁺) 259.0, found 258.9.

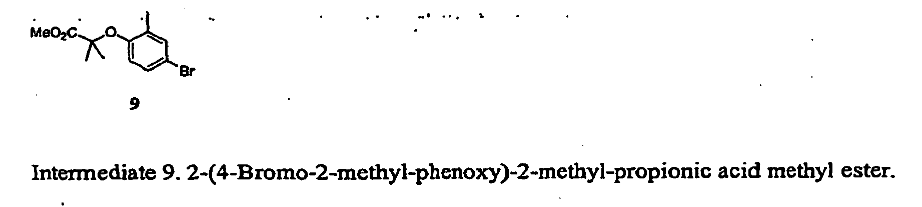

Following the procedure of Intermediate 8, except substituting α,α-dimethyl-methyl-bromoacetate for methyl-bromoacetate and heating to reflux in step B, the title compound is prepared as a clear liquid: ¹H-NMR (400MHz, CDCl₃) δ = 7.27 (d, J = 2.5 Hz, 1H), 7.14 (dd, J = 2.5 Hz, J = 8.7 Hz, 1H), 6.50 (d, J = 8.7 Hz, 1H), 2.20 (s, 3H), 1.58 (s, 6H). MS calcd. for C₁₂H₁₆BrO₃ (M+H⁺) 287.0, found 287.0.

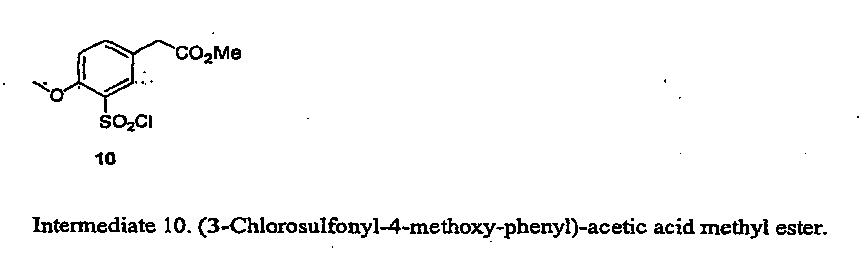

The title compound is prepared following the procedure described in the art.

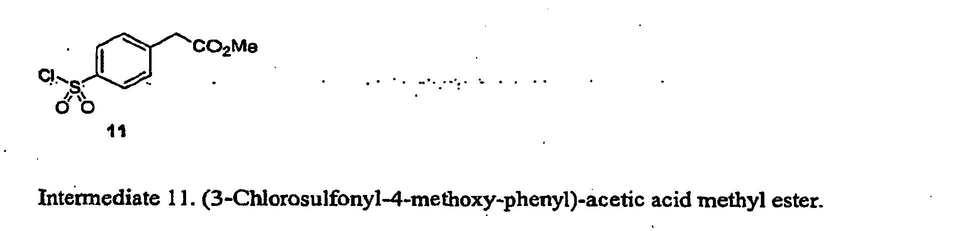

The title compound is prepared following the procedure described in the art.

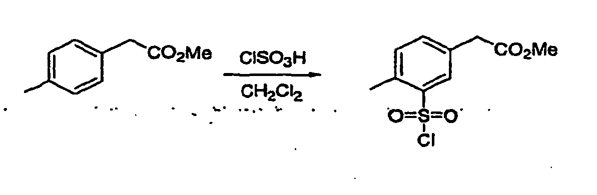

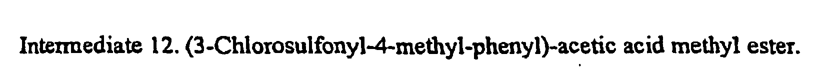

p-Tolyl-acetic acid methyl ester (1.0 g, 6.09 mmol) is dissolved in dichloromethane (4 mL) and cooled to 0°C. Chlorosulfonic acid (10 mL) is added under stirring over a period of 1 h. The mixture is warmed to room temperature and stirred for 1h at this temperature. The reaction mixture is diluted with EtOAc and washed with saturated Na₂CO₃ and brine. The organic layer is dried (MgSO₄), filtered and concentrated to give crude product, which is purified by silic gel chromatography with EtOAc/hexane to give the title product (677 mg, 42%) as an oil: ¹H-NMR (400MHz, CDCl₃) δ = 7.89 (d, J=1.6 Hz, 1H), 7.48 (dd, J = 1.6 Hz, J=7.6 Hz, 1H), 7.32 (d, J=7.6 Hz, 1H), 3.66 (s, 3H), 3.63 (s, 2H), 2.70 (s, 3H); MS calcd. for C₁₀H₁₁O₄S(M-Cl⁺) 227.04, found 227.00.

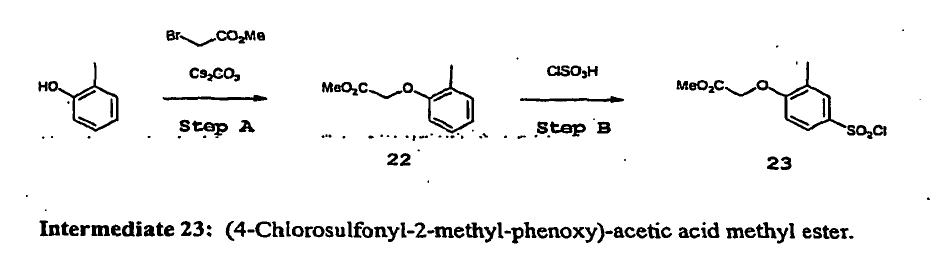

**Step A:** o-Cresol (10.0 g, 0.092 mmol) is dissolved in dry DMF (100 mL). Bromoacetic acid methyl ester (15.0 g, 0.098 mmol) and cesium carbonate (40.0 g, 0.123 mmol) are added. The reaction is kept stirring at room temperature for 3 h. Water is added and the reaction is extracted three times with ethyl acetate. The organic phase is washed with brine and dried with MgSO₄. The solvent is evaporated to give crude product 22. MS calcd. for C₁₀H₁₃O₃ (M+H⁺) 181.08, found 181.10.

**Step B:** A round bottom flask is charged with o-tolyloxy-acetic acid methyl ester 22 (5.0 g, 27.8 mmol). Chlorosulfonic acid (13.05 g, 112.0 mmol) is added at room temperature over 5 min. The reaction mixture is poured onto ice, stirred for another 5 min. Then it is filtered, the residue dissolved in DCM and washed with water three times. The organic phase is washed with saturated NaHCO₃ and brine and dried by MgSO₄. The solvent is evaporated. The crude product is purified by silica gel chromatography (ethyl acetate/hexane: 0-30%) to give 23 (4.8 g, 15.6 mmol, yield 78%) as a white solid: ¹H-NMR (400 MHz, CDCl₃) δ = 7.78 (m, 2H), 6.72 (d, *J* = 9.2 Hz, 1H), 4.71 (s, 2H), 3.76 (s, 3H), 2.30 (s, 3H). MS calcd. for C₁₀H₁₁ O₅S (M-Cl⁺) 243.0, found 243.0.

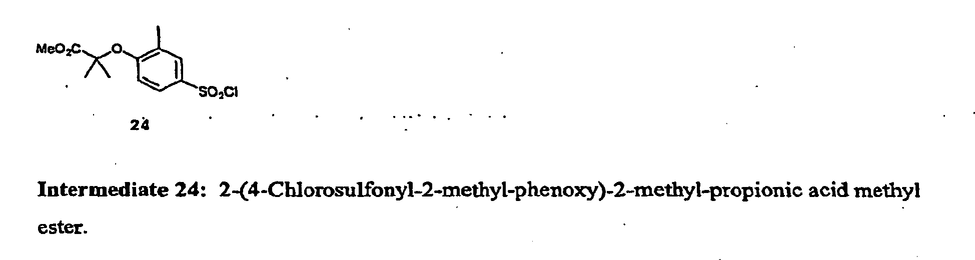

Following the procedure for Intermediate 23, except substituting 2-bromo-2-methyl-propionic acid methyl ester for bromoacetic acid methyl ester, the title compound is prepared as a white solid: ¹H-NMR (400 MHz, CDCl₃) δ = 7.82 (d, J = 1.6 Hz, 1H), 7.77 (dd, *J* = 2.8 Hz, 8.8 Hz, 1H), 6.64 (d, *J* = 8.8 Hz, 1H), 3.77 (s, 3H), 2.31 (s, 3H), 1.70(s, 6H). MS calcd. for C₁₂H₁₆ClO₅S (M+H⁺) 307.03, found 307.00.

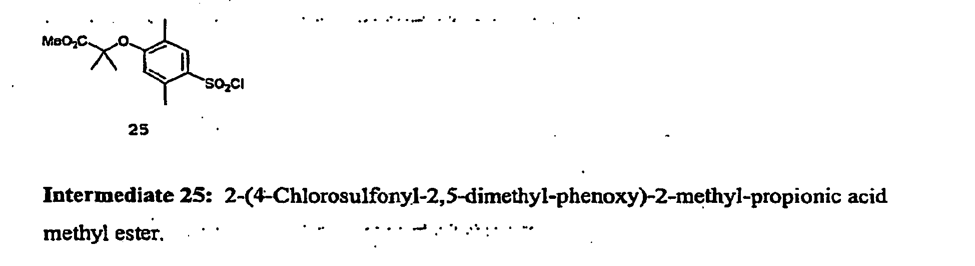

Following the procedure for Intermediate 23, except using 2-promo-2-methyl-propionic acid methyl ester and 2,5-dimethylphenol, the title compound is prepared as a white solid: ¹H-NMR (400 MHz, CDCl₃) δ = 7.51 (s, 1H), 6.31 (s, 1H), 3.71 (s, 3H), 2.40 (s, 3H), 2.10 (s, 3H), 1.51 (s, 6H). MS calcd. for C₁₃H₁₈ClO₅S (M+H⁺) 320.0, found 320.0.

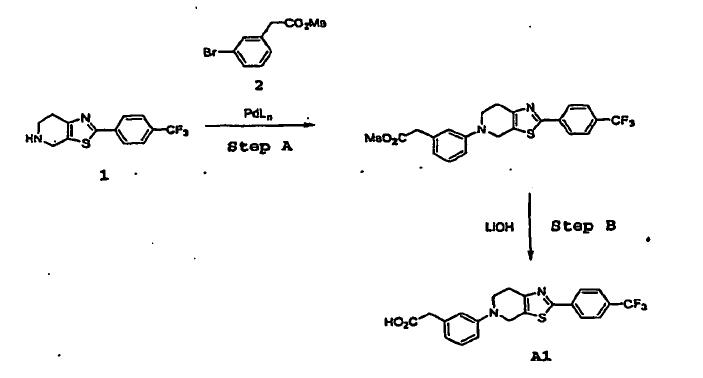

### Example A1

### {3-[2-(4-Trifluoromethyl-phenyl)-6,7-dihydro-4H-thiazolo{5,4-c]pyridin-5-yl]-phenyl}-acetic acid.

**Step A:** A flame-dried, sealed tube is charged with 2-(4-Trifluoromethylphenyl)-4,5,6,7-tetrahydro-thiazolo[5,4-c]pyridine 1 (20 mg, 0.07 mmol), (3-bromo-phenyl)-acetic acid methyl ester 16 (24 mg, 0.11 mmol), (tBu)₃PHBF₃ (2 mg, 0,01 mmol) and Cs₂CO₃ (46 mg, 0.14 mmol). 1,4-Dioxane (0.5 mL) is added and the tube is purged with argon. Then Pd₂(dba)₃ (3 mg, 0.003 mmol) is added and the mixture is heated at 120°C overnight. The mixture containing crude {3-[2-(4-Trifluoromethyl-phenyl)-6,7-dihydro-4H-thiazolo[5,4-c]pyridin-5-yl]-phenyl}-acetic acid methyl ester is used without further purification in Step B.

**Step B:** To the reaction mixture of Step A is added THF (1.5 mL), a solution of 1 M LiOH in H₂O (0.7 mL) is added and the mixture is stirred for 12 h at room temperature. The mixture is acidified with 1 M HCl (1 mL) and extracted with DCM twice. The organic layer is washed with brine, dried (MSO₄), filtered, concentrated and purified on reverse phase HPLC (H₂O/MeCN gradient) to afford the title compound (13 mg, 44% over 2 steps) as a colourless glass: ¹H-NMR (400MHz, CDCl₃) δ = 8.00 (d, J = 8.2 Hz, 2H), 7.69 (d, J = 8.2 Hz, 2H), 7.27 (m, 1H), 6.97 (m, 2H), 6.87 (d, J = 7.5 Hz, 1H), 4.55 (s, 2H), 3.74 (t, J = 5.7 Hz, 2H), 3.63 (s, 2H), 3.08 (t, J = 5.4 Hz, 2H). MS calculated for C₂₁H₁₈F₃N₂O₂S (M+H⁺) 419.1, found 419.0.

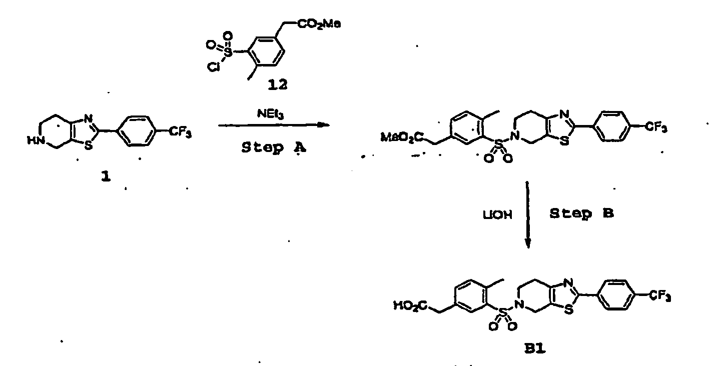

### Example B1

### {4-Methyl-3-[2-(4-trifluoromethyl-phenyl)-6,7-dihydro-4H-thiazolo[5,4-c]pyridine-5-sulfonyl]-phenyl} -acetic acid.

**Step A:** The 2-(4-trifluoromethyl-phenyl)-4,5,6,7-tetrahydro-thiazolo[5,4-c]pyridine **1** (14 mg, 0.05 mmol) is dissolved in DCM (0.5 mL). Triethylamine (14 uL, 0.10 mmol) and (3-chlorosulfonyl-4-methyl-phenyl)-acetic acid methyl ester **12** (16 mg, 0.06 mmol) are added successively and the mixture is stirred at room temperature for 8 h. The solvent is removed in vacuo to afford crude {4-methyl-3-[2-(4-trifluoromethyl-phenyl)-6,7-dihydro-4H-thiazolo[5,4-c]pyridine-5-sulfonyl]-phenyl}-acetic acid methyl ester which is used without further purification in Step B.
**Step B:** The crude {4-methyl-3-[2-(4-trifluoromethyl-phenyl)-6,7-dihydro-4H-thiazolo[5,4-c]pyridine-5-sulfonyl]-phenyl}-acetic acid methyl ester is dissolved in THF (1 mL), a solution of 1 M LiOH in H₂O (0.6 mL) is added and the mixture is stirred for 12h at room temperature. The mixture is acidified with 1 M HCl (0.8 mL) and extracted with DCM twice. The organic layer is washed with brine, dried (MgSO₄), filtered, concentrated and purified on reverse phase HPLC (H₂O/MeCN gradient) to afford the title compound (17 mg, 68% over 2 steps) as a colourless solid: ¹H-NMR (400MHz, CDCl₃) δ = 7.97 (d, J = 8.2 Hz, 2H), 7.92 (d, J = 1.6 Hz, 1H), 7.68 (d, J = 8.2 Hz, 2H), 7.41 (dd, J = 1.6 Hz, J = 7.8 Hz, 1H), 7.30 (d, J = 7.8 Hz, 1H), 4.57 (s, 2H), 3.70 (s, 2H), 3.65 (t, J = 5.8 Hz, 2H), 3.00 (t, J = 5.7 Hz; 2H), 2.60 (s, 3H). MS calculated for C₂₂H₂₀F₃N₂O₅S₂ (M+H⁺) 497.1, found 497.1.

By repeating the procedures described in the above examples, using appropriate starting materials, the following compounds of Formula I, as identified in Table 1, are obtained.

**Table 1**

| **Compound Number** | **Compound Structure** | **Physical Data** **¹H NMR and/or MS (m/z)** |
|---|---|---|
| A2 | 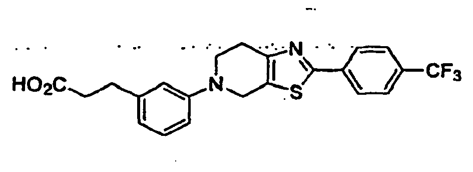 | ¹H-NMR (400MHz, CDCl₃) δ = 8.00 (d, J = 8.3 Hz, 2H), 7.69 (d, J = 8.3 Hz, 2H), 7.26 (t, J = 7.6 Hz, 1H), 6.97 (m, 2H), 6.87 (d, J = 7.6 Hz, 1H), 4.59 (s, 2H), 3.76 (t, J = 5.8 Hz, 2H), 3.09 (t, J = 5.7 Hz, 2H), 2.94 (t, J = 7.6 Hz, 2H), 2.68 (t, J = 7.6 Hz, 2H). MS calculated for C₂₂H₂₀F₃N₂O₂S (M+H⁺) 433.1, found 433.1. |
| A3 | 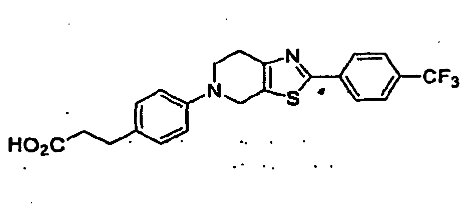 | ¹H-NMR (400MHz, CDCl₃) δ = 8.01 (d, J = 8.5 Hz, 2H), 7.68 (d, J = 8.5 Hz, 2H), 7.15 (d, J = 8.5 Hz, 2H), 6.98 (d, J = 8.5 Hz, 2H), 4.51 (s, 2H), 3.70 (t, J = 5.7 Hz, 2H). 3.05 (t, J = 5.7 Hz, 2H), 2.90 (t, J = 7.6 Hz, 2H), 2.65 (t, J = 7.6 Hz, 2H). MS calculated for C₂₂H₂₀F₃N₂O₂S (M+H⁺) 433.1, found 433.1. |
| A4 | 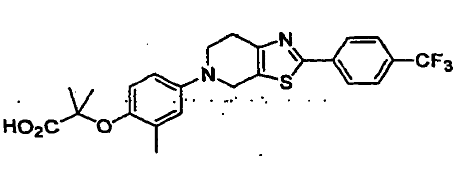 | MS calculated for C₂₄H₂₄F₃N₂O₃S (M+H⁺) 477.1, found 477.1. |
| B2 | 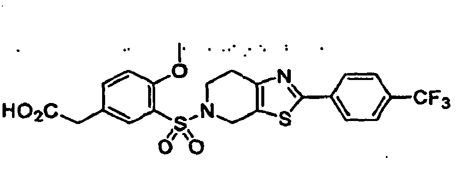 | ¹H-NMR (400MHz, CDCl₃) δ = 7.97 (d, J = 8.2 Hz, 2H), 7.90 (d, J = 2.2 Hz, I H), 7.67 (d, J = 8.2 Hz, 2H), 7.45 (dd, J = 2.2 Hz, J = 8.5 Hz, 1H), 6.92 (d, J = 8.5 Hz, 1H), 4.72 (s, 2H), 3.78 (s, 3H), 3.72 (t, J = 5.8 Hz, 2H), 3.66 (s, 2H), 2.84 (t, J = 5.7 Hz, 2H). MS calculated for C₂₂H₂₀F₃N₂O₅S₂ (M+H⁺) 513.0, found 513.0. |
| B3 | 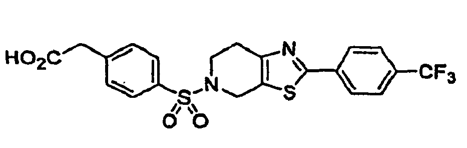 | 1H-NMR (400MHz, CDCl₃) δ= 7.89 (d, J = 8.1 Hz, 2H), 7.73 (m, 2H), 7.60 (d, J = 8.1 Hz, 2H), 7.40 (m, 2H), 4.39 (t, J = 5.4 Hz, 1H), 3.64 (s, 2H), 3.48 (t, J = 5.8 Hz, 2H), 2.93 (t, J = 5.9 Hz, 2H). MS calculated for C₂₁H₁₈F₃N₂O₄S₂ (M+H⁺) 483.1, found 483.0. |
| B4 | 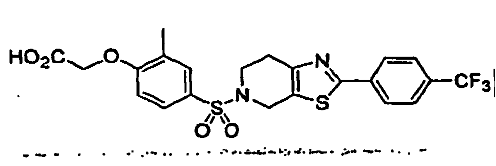 | ¹H-NMR (400MHz, CDCl₃) δ= 7.90 (d, J = 8.2 Hz, 2H), 7.62-7.57 (m, 4H), 6.73 (d, J = 8.1 Hz, 1H), 4.68 (s, 2H), 4.38 (s, 2H), 3.47 (t, J = 5.8 Hz, 2H), 2.91 (m, 2H), 2.25 (s, 3H). MS calculated for C₂₂H₂₀F₃N₂O₅S₂ (M+H⁺) 513.1, found 513.0. |
| B5 | 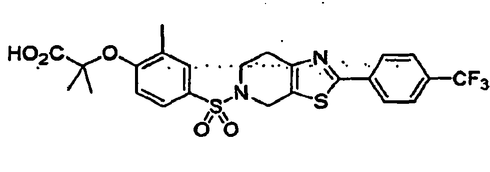 | ¹H-NMR (400MHz, CDCl₃) δ = 7.94 (d, J = 8.2 Hz, 2H), 7.67 (d, J = 8.2 Hz, 2H), 7.63 (d, J = 2.1 Hz, 1H), 7.57 (dd, J = 2.1 Hz, J = 8.6 Hz, 1H), 6.76 (d, J = 8.6 Hz, 1H), 4.46 (s, 2H), 3.54 (t, J = 5.8 Hz, 2H), 2.94 (t, J = 5.8 Hz, 2H), 2.26 (s, 3H), 1.68 (s, 6H). MS calculated for C₂₄H₂₄F₃N₂O₅S₂ (M+H⁺) 541.1, found 541.1. |
| B6 | 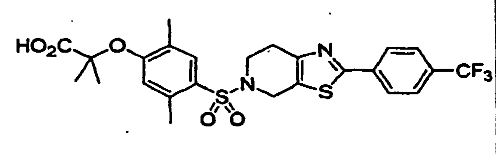 | ¹H-NMR (400MHz, CDCl₃) δ = 7.96 (d, J = 8.2 Hz, 2H), 7.80 (s, 1H), 7.68 (d, J = 8.2 Hz, 2H), 6.58 (s, 1H), 4.54 (s, 2H), 3.62 (t, J = 5.8 Hz, 2H), 2.94 (t, J = 5.8 Hz, 2H), 2.48 (s, 3H), 2.24 (s, 3H), 1.68 (s, 6H). MS calculated for C₂₅H₂₆F₃N₂O₅S₂ (M+H⁺) 555.1, found 555.2. |

### Transcriptional Assay

Transfection assays are used to assess the ability of compounds of the invention to modulate the transcriptional activity of the PPARs. Briefly, expression vectors for chimeric proteins containing the DNA binding domain of yeast GAI4 fused to the ligand-binding domain (LBD) of either PPARδ, PPARα or PPARγ are introduced via transient transfection into mammalian cells, together with a reporter plasmid where the luciferase gene is under the control of a GAL4 binding site. Upon exposure to a PPAR modulator, PPAR transcriptional activity varies, and this can be monitored by changes in luciferase levels. If transfected cells are exposed to a PPAR agonist, PPAR-dependent transcriptional activity increases and luciferase levels rise.

293T human embryonic kidney cells (8×10⁶) are seeded in a 175cm² flask a day prior to the start of the experiment in 10% FBS, 1% Penicillin/Streptomycin/Fungizome, DMEM Media. The cells are harvested by washing with PBS (30ml) and then dissociating using trypsin (0.05%; 3ml). The trypsin is inactivated by the addition of assay media (DMEM, CA-dextran fetal bovine serum (5%). The cells are spun down and resuspended to 170,000cells/ml. A Transfection mixture of GAL4-PPAR LBD expression plasmid (1µg), UAS-luciferase reporter plasmid (1 µg), Fugene (3:1 ratio; 6µL) and serum-free media (200µL) was prepared and incubated for 15-40 minutes at room temperature. Transfection mixtures are added to the cells to give 0.16M cells/mL, and cells (50µl/well) are then plated into 384 white, solid-bottom, TC-treated plates. The cells are further incubated at 37°C, 5.0% CO₂ for 5-7 hours. A 12-point series of dilutions (3 fold serial dilutions) are prepared for each test compound in DMSO with a starting compound concentration of 10µM. Test compound (500nl) is added to each well of cells in the assay plate and the cells are incubated at 37°C, 5.0% CO₂ for 18-24 hours. The cell lysis/luciferase assay buffer, Bright-Glo^{™} (25%; 25µl; Promega), is added to each well. After a further incubation for 5 minutes at room temperature, the luciferase activity is measured.

Raw luminescence values are normalized by dividing them by the value of the DMSO control present on each plate. Normalized data is analyzed and dose-response curves are fitted using Prizm graph fitting program. EC50 is defined as the concentration at which the compound elicits a response that is half way between the maximum and minimum values. Relative efficacy (or percent efficacy) is calculated by comparison of the response elicited by the compound with the maximum value obtained for a reference PPAR modulator.

Compounds of Formula I, in free form or in pharmaceutically acceptable salt farm, exhibit valuable pharmacological properties, for example, as indicated by the in vitro tests described in this application. Compounds of the invention preferably have an EC50 for PPARδ and/or PPARα and/or PPARγ, of less than 5µM, more preferably less than 1 µM, more preferably less than 500nm, more preferably less than 100nm. Compounds of the invention preferably have an EC50 for PPARδ that is less than or equal to PPARα which in turn has an EC50 that is at least 10-fold less than PPARγ.

It is understood that the examples and embodiments described herein are for illustrative purposes only.

## Claims

1. A compound of Formula I: 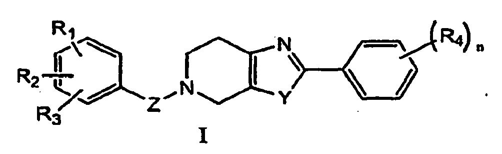 in which:
n is selected from 0, 1 and 2;
R₁ is selected from -XCO₂R₁₃ and -OCR₁₁R₁₂XCO₂R₁₃; wherein X is selected from a bond and C₁₋₄alkylene; R₁₁ and R₁₂ are independently selected from hydrogen, C₁₋₄alkyl and C₁₋₄alkoxy; or R₁₁ and R₁₂ together with the carbon atom to which R₁₁ and R₁₂ are attached form C₃₋₁₂cycloalkyl; and R₁₃ is selected from hydrogen and C₁₋₆alkyl;
R₂ and R₃ are independently selected from hydrogen, halo, C₁₋₆alkyl, halo-substituted C₁₋₆alkyl, C₁₋₆alkoxy and halo-substituted-C₁₋₆alkoxy;
Z is selected from a bond and -S(O)₀₋₂-;
Y is selected from O and S;
R₄ is selected from hydrogen, halo, C₁₋₆alkyl, halo-substituted-C₁₋₆alkyl, C₁₋₆alkoxy and halo-substituted-C ₁-₆alkoxy; and the pharmaceutically acceptable salts, hydrates and solvates thereof.

2. The compound of claim 1 in which:
n is selected from 0 and 1;
R₁ is selected from XCO₂R₁₃ and -OCR₁₁R₁₂XCO₂R₁₃; wherein X is selected from a bond and C₁₋₄alkylene; R₁₁ and R₁₂ are independently selected from hydrogen, C₁₋₄alkyl and C₁₋₄alkoxy; or R₁₁ and R₁₂ together with the carbon atom to which R₁₁ and R₁₂ are attached form C₃₋₁₂cycloalkyl; and R₁₃ is selected from hydrogen and C₁₋₆alkyl;
R₂ and R₃ are independently selected from hydrogen, halo, C₁₋₆alkyl, halo-substituted C₁₋₆alkyl, C₁₋₆alkoxy and halo-substituted-C₁₋₆alkoxy;
Z is selected from a bond and -S(O)₀₋₂-;
Y is selected from O and S; and
R₄ is halo-substituted-C₁₋₆alkyl.

3. The compound of claim 2 in which: R₁ is selected from -CH₂CO₂H, - (CH₂)₂CO₂H, -OC(CH₂)₂CO₂H and -OCH₂CO₂H.

4. The compound of claim 3 in which R₂ and R₃ are independently selected from hydrogen, methyl and methoxy; and R₄ is trifluoromethyl.

5. The compound of claim 4 selected from: {3-[2-(4-Trifluoromethyl-phenyl)-6,7-dihydro-4H-thiazolo[5,4-c]pyridin-5-yl]-phenyl}-acetic acid; 3-{3-[2-(4-Trifluoromethylphenyl)-6,7-dihydro-4H-thiazolo[5,4-c]pyridin-5-yl]-phenyl-propionic acid; 3-{4-[2-(4-Trifluoromethyl-phenyl)-6,7-dihydro-4H-thiazolo[5,4-c]pyridin-5-yl]-phenyl}-propionic acid; 2-Methyl-2-{2-methyl-4-[2-(4-trifluoromethyl-phenyl)-6,7-dihydro-4H-thiazolo[5,4-c]pyridin-5-yl]-phenoxy}-propionic acid; {4-Methyl-3-[2-(4-trifluoromethyl-phenyl)-6,7-dihydro-4H-thiazolo[5,4-c]pyridine-5-sulfonyl]-phenyl}-acetic acid; {4-Methoxy-3-[2-(4-trifluoromethyl-phenyl)-6,7-dihydro-4H-thiazolo[5,4-c]pyridine-5-sulfonyl]-phenyl}-acetic acid; {4-[2-(4-Trifluoromethyl-phenyl)-6,7-dihydro-4H-thiazolo[5,4-c]pyridine-5-sulfonyl]-phenyl}-acetic acid; {2-Methyl-4-[2-(4-trifluoromethyl-phenyl)-6,7-dihydro-4H-thiazolo[5,4-c]pyridine-5-sulfonyl]-phenoxy}-acetic acid; 2-Methyl-2-{2-methyl-4-[2-(4-trifluoromethyl-phenyl)-6,7dihydro-4H-thiazolo[5,4-c]pyridine-5-sulfonyl]-phenoxy}-propionic acid; and 2-{2,5-Dimethyl-4-[2-(4-trifluoromethyl-phenyl)-6,7-dihydro-4H-thiazolo[5,4-c]pyridine-5-sulfonyl)-phenoxy)-2-methyl-propionic acid.

6. The use of a compound according to any of claims 1 to 5 in the manufacture of a medicament for treating a disease in an animal in which PPAR activity contributes to the pathology and/or symptomology of the disease.

7. The use of claim 6 in which the PPAR activity is at least one PPAR selected from PPARα, PPARδ and PPARγ.

8. The use of claim 7 in which the PPAR activity is both PPARα and PPARδ.

9. A pharmaceutical composition comprising a therapeutically effective amount of a compound of any of claim 1 to 5 in combination with one or more pharmaceutically acceptable excipients.

10. A pharmaceutical combination, especially a pharmaceutical composition, comprising: 1) a compound of any of claims 1 to 5 or a pharmaceutical acceptable salt thereof; and 2) at least one active ingredient selected from:
a) anti-diabetic agents such as insulin, insulin derivatives and mimetics; insulin secretagogues such as the sulfonylureas, e.g., Glipizide, glyburide and Amaryl; insulinotropic sulfonylurea receptor ligands such as meglitinides, e.g., nateglinide and repaglinide; insulin sensitizer such as protein tyrosine phosphatase-1B (PTP-1B) inhibitors such as PTP-112; GSK3 (glycogen synthase kinase-3) inhibitors such as Sub-517955, SB-4195052, SB-216763, NN-57-05441 and NN-57-05445; RXR ligands such as GW-0791 and AGN-194204; sodium-dependent glucose co-transporter inhibitors such as T-1095; glycogen phosphorylase A inhibitors such as BAY R3401; biguanides such as metformin; alpha-glucosidase inhibitors such as acarbose; GLP-1 (glucagon like peptide-1), GLP-1 analogs such as Exendin-4 and GLP-1 mimetics; dipeptidyl peptidase IV inhibitors such as DPP728, vildagliptin, MK-0431, saxagliptin, GSK23A ; an AGE breaker, a thiazolidone derivative (glitazone) such as pioglitazone, rosiglitazone, or (R)-1-{4-[5-methyl-2-(4-trifluoromethylphenyl)-oxazol-4-ylmethoxy]-benzenesulfonyl}-2,3-dihydro-1H-indole-2-carboxylic acid, a non-glitazone type PPARγ agonist e.g. GI-262570;
b) hypolipidemic agents such as 3-hydroxy-3-methyl-glutaryl coenzyme A (HMG-CoA) réductase inhibitors, e.g., lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (farnesoid X receptor) and LXR (liver X receptor) ligands; cholestyramine; fibrates; nicotinic acid and aspirin;
c) an anti-obesity agent or appetite regulating agent such as phentermine, leptin, bromocriptine; dexamphetamine, amphetamine,fenfluramine, dexfenfluramine, sibutramine, orlistat, dexfenfluramine, mazindol, phentermine, phendimetrazine, diethylpropion, fluoxetine, bupropion, topiramate, diethylpropion, benzphetamine, phenylpropanolamine or ecopipam, ephedrine, pseudoephedrine or cannabinoid receptor antagonists;
d) anti-hypertensive agents, e.g., loop diuretics such as ethacrynic acid, furosemide and torsemide; diuretics such as thiazide derivatives, chlorithiazide, hydrochlorothiazide, amildride; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors e.g. thiorphan, terteo-thiorphan, SQ29072; ECE inhibitors e.g. SLV306; ACE/NEP inhibitors such as omapatrilat, sampatrilat and fasidotril; angiotensin **II** antagonists such as candesartan, eprosartan, irbesartan, losartan, telmisartan and valsartan, in particular valsartan; renin inhibitors such as aliskiren, terlakiren, ditekiren, RO 66-1132, RO-66-1168; β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; and aldosterone synthase inhibitors;
e) a HDL increasing compound;
f) a cholesterol absorption modulator such as Zetia® and KT6-971;
g) Apo-A1 analogues and mimetics;
h) thrombin inhibitors such as Ximelagatran;
i) aldosterone inhibitors such as anastrazole, fadrazole, eplerenone;
j) Inhibitors of platelet aggregation such as aspirin, clopidogrel bisulfate;
k) estrogen, testosterone, a selective estrogen receptor modulator, a selective androgen receptor modulator;
l) a chemotherapeutic agent such as aromatase inhibitors e.g. femara, anti-estrogens, topoisomerase I inhibitors, topoisomerase II inhibitors, microtubule active agents, alkylating agents, antineoplastic antimetabolibes, platin compounds, compounds decreasing the protein kinase activity such as a PDGF receptor tyrosine kinase inhibitor preferably Imatinib or 4-Methyl-N-[3-(4-methyl-imidazol-1-yl)-5 trifluoromethyl-phenyl]-3-(4-pyridin-3-yl-pyrimidin-2-ylamino)-benzamide; and
m) an agent interacting with a 5-HT₃ receptor and/or an agent interacting with 5-HT₄ receptor such as tegaserod, tegaserod hydrogen maleate, cisapride, cilansetron;
or, in each case a pharmaceutically acceptable salt thereof; and optionally a pharmaceutically acceptable carrier.

11. A pharmaceutical composition according to claim 9 or a combination according to claim 10, for use in a method for the treatment or prevention of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome-X.

12. A pharmaceutical composition according to claim 9 or a combination according to claim 10, for use in a method for the treatment or prevention of HIV wasting syndrome, long term critical illness, decreased muscle mass and/or muscle strength decreased lean body mass, maintenance of muscle strength and function in the elderly, diminished muscle endurance and muscle function, and frailty in the elderly.

13. A compound according to any of claims 1 to 5, or a pharmaceutical composition according to claim 9 or a combination according to claim 10, for use in method of treatment.

14. Use of a compound according to any of claims 1 to 5, or a pharmaceutical composition according to claim 9 or a combination according to claim 10, for the manufacture of a medicament for the treatment or prevention of dyslipidemia, hyperlipidemia, hypercholesteremia, atherosclerosis, hypertriglyceridemia, heart failure, myocardial infarction, vascular diseases, cardiovascular diseases, hypertension, obesity, inflammation, arthritis, cancer, Alzheimer's disease, skin disorders, respiratory diseases, ophthalmic disorders, inflammatory bowel diseases, IBDs (irritable bowel disease), ulcerative colitis, Crohn's disease, conditions in which impaired glucose tolerance, hyperglycemia and insulin resistance are implicated, such as type-1 and type-2 diabetes, Impaired Glucose Metabolism (IGM), Impaired Glucose Tolerance (IGT), Impaired Fasting Glucose (IFG), and Syndrome-X.

15. Use of a compound according to any of claims 1 to 5, or a pharmaceutical composition according to claim 9 or a combination according to claim 10, for the manufacture of a medicament for the treatment or prevention of HIV wasting syndrome, long term critical illness, decreased muscle mass and/or muscle strength decreased lean body mass, maintenance of muscle strength and function in the elderly, diminished muscle endurance and muscle function, and frailty in the elderly.

## Patentansprüche

1. Verbindung der Formel I: 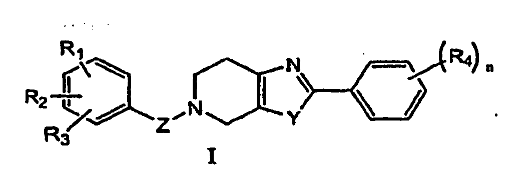 worin:
n aus 0, 1 und 2 ausgewählt ist;
R₁ aus -XCO₂R₁₃ und -OCR₁₁R₁₂XCO₂R₁₃ ausgewählt ist, worin X aus einer Bindung und C₁₋₄-Alkylen ausgewählt ist; R₁₁ und R₁₂ unabhängig voneinander aus Wasserstoff, C₁₋₄-Alkyl und C₁₋₄-Alkoxy ausgewählt sind oder R₁₁ und R₁₂ gemeinsam mit dem Kohlenstoffatom, an das R₁₁ und R₁₂ gebunden sind, C₃₋₁₂-Cycloalkyl bilden; und R₁₃ aus Wasserstoff und C₁₋₆-Alkyl ausgewählt ist;
R₂ und R₃ unabhängig voneinander aus Wasserstoff, Halogen, C₁₋₆-Alkyl, halogensubstituiertem C₁₋₆-Alkyl, C₁₋₆-Alkoxy und halogensubstituiertem C₁₋₆-Alkoxy ausgewählt sind;
Z aus einer Bindung und -S(O)₀₋₂- ausgewählt ist;
Y aus O und S ausgewählt ist;
R₄ aus Wasserstoff, Halogen, C₁₋₆-Alkyl, halogensubstituiertem C₁₋₆-Alkyl, C₁₋₆-Alkoxy und halogensubstituiertem C₁₋₆-Alkoxy ausgewählt ist; sowie die pharmazeutisch annehmbaren Salze, Hydrate und Solvate davon.

2. Verbindung nach Anspruch 1, worin:
n aus 0 und 1 ausgewählt ist;
R₁ aus -CO₂R₁₃ und -OCR₁₁R₁₂XCO₂R₁₃ ausgewählt ist, worin X aus einer Bindung und C₁₋₄-Alkylen ausgewählt ist; R₁₁ und R₁₂ unabhängig voneinander aus Wasserstoff, C₁₋₄-Alkyl und C₁₋₄-Alkoxy ausgewählt sind oder R₁₁ und R₁₂ gemeinsam mit dem Kohlenstoffatom, an das R₁₁ und R₁₂ gebunden sind, C₃₋₁₂-Cycloalkyl bilden; und R₁₃aus Wasserstoff und C₁₋₆-Alkyl ausgewählt ist;
R₂ und R₃ unabhängig voneinander aus Wasserstoff, Halogen, C₁₋₆-Alkyl, halogensubstituiertem C₁₋₆-Alkyl, C₁₋₆-Alkoxy und halogensubstituiertem C₁₋₆-Alkoxy ausgewählt sind;
Z aus einer Bindung und -S(O)₀₋₂- ausgewählt ist;
Y aus O und S ausgewählt ist und
R₄ halogensubstituiertes C₁₋₆-Alkyl ist.

3. Verbindung nach Anspruch 2, worin R₁ aus -CH₂CO₂H, -(CH₂)₂CO₂H, -OC(CH₂)₂CO₂H und -OCH₂CO₂H ausgewählt ist.

4. Verbindung nach Anspruch 3, worin R₂ und R₃ unabhängig voneinander aus Wasserstoff, Methyl und Methoxy ausgewählt sind und R₄ Trifluormethyl ist.

5. Verbindung nach Anspruch 4, die aus folgenden ausgewählt ist: {3-[2-(4-Trifluormethylphenyl)-6,7-dihydro-4H-thiazolo[5,4-c]pyridin-5-yl]phenyl}essigsäure; 3-{3-[2-(4-Trifluormethylphenyl)-6,7-dihydro-4H-thiazolo[5,4-c]pyridin-5-yl]phenyl}propionsäure; 3-{4-[2-(4-Trifluormethylphenyl)-6,7-dihydro-4H-thiazolo[5,4-c]pyridin-5-yl]phenyl}propionsäure; 2-Methyl-2-{2-methyl-4-[2-(4-trifluormethylphenyl)-6,7-dihydro-4H-thiazolo[5,4-c]pyridin-5-yl]phenoxy}propionsäure; {4-Methyl-3-[2-(4-trifluormethylphenyl)-6,7-dihydro-4H-thiazolo[5,4-c]pyridin-5-sulfonyl]phenyl}essigsäure; {4-Methoxy-3-[2-(4-trifluormethylphenyl)-6,7-dihydro-4H-thiazolo[5,4-c-]pyridin-5-sulfonyl]phenyl}-essigsäure; {4-[2-(4-Trifluormethylphenyl)-6,7-dihydro-4H-thiazolo[5,4-c]pyridin-5-sulfonyl]phenyl}essigsäure; {2-Methyl-4-[2-(4-trifluormethylphenyl)-6,7-dihydro-4H-thiazolo[5,4-c]pyridin-5-sulfonyl]phenoxy}essigsäure; 2-Methyl-2-{2-methyl-4-[2-(4-trifluormethylphenyl)-6,7-dihydro-4H-thiazolo[5,4-c]pyridin-5-sulfonyl]phenoxy}propionsäure und 2-{2,5-Dimethyl-4-[2-(4-trifluormethylphenyl)-6,7-dihydro-4H-thiazolo[5,4-c]pyridin-5-sulfonyl]phenoxy}-2-methylpropionsäure.

6. Verwendung einer Verbindung nach einem der Ansprüche 1 bis 5 zur Herstellung eines Medikaments zur Behandlung einer Erkrankung in einem Tier, bei dem die PPAR-Aktivität zu der Pathologie und/oder Symptomatik der Erkrankung beiträgt.

7. Verwendung nach Anspruch 6, wobei die PPAR-Aktivität die Aktivität von zumindest einem aus PPARα, PPARδ und PPARγ ausgewählten PPAR betrifft.

8. Verwendung nach Anspruch 7, wobei die PPAR-Aktivität sowohl PPARα als auch PPARδ betrifft.

9. Pharmazeutische Zusammensetzung, umfassend eine therapeutisch wirksame Menge einer Verbindung nach einem der Ansprüche 1 bis 5 in Kombination mit einem oder mehreren pharmazeutisch annehmbaren Exzipienten.

10. Pharmazeutische Kombination, insbesondere eine pharmazeutische Zusammensetzung, die Folgendes umfasst: 1) eine Verbindung nach einem der Ansprüche 1 bis 5 oder ein pharmazeutisch annehmbares Salz davon und 2) zumindest einen aus Folgendem ausgewählten Wirkstoff:
a) Anti-Diabetes-Mittel, wie z.B. Insulin, Insulinderivate und -mimetika; Insulinsekretagoga, wie z.B. Sulfonylharnstoffe, z.B. Glipizid, Glyburid und Amaryl; insulinotrope Sulfonylharnstoffrezeptorliganden, wie z.B. Meglitinide, z.B. Nateglinid und Repaglinid; Insulinsensibilisierungsmittel, wie z.B. Proteintyrosinphosphatase-1B- (PTP-1B-) Hemmer, z.B. PTP-112; GSK3- (Glykogensynthasekinase-3-) Hemmer, wie z.B. SB-517955, SB-4195052, SB-216763, NN-57-05441 und NN-57-05445; RXR-Liganden, wie z.B. GW-0791 und AGN-194204; natriumabhängige Glucose-Co-Transporterinhibitoren, wie z.B. T-1095; Glykogenphosphorylase-A-Hemmer, wie z.B. BAY R3401; Biguanide, wie z.B. Metformin; α-Glucosidasehemmer, wie z.B. Ascarbose; GLP-1 (Glucagon-ähnliches Peptid-1), GLP-1-Analoga, wie z.B. Exendin-4, und GLP-1-Mimetika; Dipeptidylpeptidase-IV-Hemmer, wie z.B. DPP728, Vildagliptin, MK-0431, Saxagliptin, GSK23A; einen AGE-Spalter; ein Thiazolidonderivat (Glitazon), wie z.B. Pioglitazon, Rosiglitazon oder (R)-1-{4-[5-Methyl-2-(4-trifluormethylphenyl)oxazol-4-ylmethoxy]benzolsulfonyl}-2,3-dihydro-1H-indol-2-carbonsäure, einen PPARy-Agonisten vom Nicht-Glitazon-Typ, wie z.B. GI-262570;
b) hypolipidämische Mittel, wie z.B. 3-Hydroxy-3-methylglutarylcoenzym-A- (HMG-CoA-) Reductasehemmer, z.B. Lovastatin, Pitavastatin, Simvastatin, Pravastatin, Cerivastatin, Mevastatin, Velostatin, Fluvastatin, Dalvastatin, Atorvastatin, Rosuvastatin und Rivastatin; Squalensynthasehemmer; FXR- (Farnesoid-X-Rezeptor-) und LXR-(Leber-X-Rezeptor-) Liganden; Cholestyramin; Fibrate; Nicotinsäure und Aspirin;
c) Anti-Obesitätsmittel oder appetitregulierende Mittel, wie z.B. Phentermin, Leptin, Bromcriptin, Dexamphetamin, Amphetamin, Fenfluramin, Dexfenfluramin, Sibutramin, Orlistat, Dexfenfluramin, Mazindol, Phentermin, Phendimetrazin, Diethylpropion, Fluoxetin, Bupropion, Topiramat, Diethylpropion, Benzphetamin, Phenylpropanolamin oder Ecopipam, Ephedrin, Pseudoephedrin oder Cannabinoidrezeptorantago-Hypertensionsmittel, wie z.B. Schleifendiuretika, wie Ethacrynsäure, Furosemid und Torsemid; Diuretika, wie Thiazidderivate, Chlorthiazid, Hydrochlorthiazid, Amilorid; Angiotensin-umwandelndes-Enzym- (ACE-) Hemmer, wie z.B. Benazepril, Captopril, Enalapril, Fosinopril, Lisinopril, Moexipril, Perinodopril, Quinapril, Ramipril und Trandolapril; Hemmer der Na-K-ATPase-Membranpumpe, wie z.B. Digoxin; Neutralendopeptidase- (NEP-) Hemmer, wie z.B. Thiorphan, terteo-Thiorphan, SQ29072; ECE-Hemmer, wie z.B. SLV306; ACE/NEP-Hemmer, wie z.B. Omapatrilat, Sampatrilat und Fasidotril; Angiotensin-II-Antagonisten, wie z.B. Candesartan, Eprosartan, Irbesartan, Losartan, Telmisartan und Valsartan, insbesondere Valsartan; Reninhemmer, wie z.B. Aliskiren, Terlakiren, Ditekiren, RO 66-1132, RO 66-1168; β-adrenerge Rezeptorblocker, wie z.B. Acebutolol, Atenolol, Betaxolol, Bisoprolol, Metoprolol, Nadolol, Propranolol, Sotalol und Timolol; inotrope Mittel, wie z.B. Digoxin, Dobutamin und Milrinon; Calciumkanal-Blocker, wie z.B. Amlodipin, Bepridil, Diltiazem, Felodipin, Nicardipin, Nimodipin, Nifedipin, Nisoldipin und Verapamil; Aldosteronrezeptorantagonisten und Aldosteronsynthasehemmer;
e) eine HDL-steigernde Verbindung;
f) einen Cholesterin-Absorptionsmodulator, wie z.B. Zetia® und KT6-971;
g) Apo-A1-Analoga und -Mimetika;
h) Thrombinhemmer, wie z.B. Ximelagatran;
i) Aldosteronhemmer, wie z.B. Anastrazol, Fadrazol, Eplerenon;
j) Hemmer der Blutplättchenaggregation, wie z.B. Aspirin, Clopidogrelbisulfat;
k) Östrogen, Testosteron, einen selektiven Östrogenrezeptormodulator, einen selektiven Androgenrezeptormodulator;
l) ein chemotherapeutisches Mittel, wie z.B. Aromatasehemmer, z.B. Femara, Anti-Östrogene, Topoisomerase-I-Hemmer, Topoisomerase-II-Hemmer, Mikrotubuli-Wirkstoffe, 'Alkylierungsmittel, antineoplastische Antimetaboliten, Platinverbindungen, Verbindungen, die die Proteinkinaseaktivität senken, wie z.B. einen PDGF-Rezeptor-Tyrosinkinasehemmer, vorzugsweise Imatinib oder 4-Methyl-N-[3-(4-methylimidazol-1-yl)-5-trifluormethylphenyl]-3-(4-pyridin-3-ylpyrimidin-2-ylamino)benzamid; und
m) ein Mittel, das mit einem 5-HT₃-Rezeptor wechselwirkt, und/oder ein Mittel, das mit einem 5-HT₄-Rezeptor wechselwirkt, wie z.B. Tegaserod, Tegaserodhydrogenmaleat, Cisaprid, Cilansetron;
oder jeweils ein pharmazeutisch annehmbares Salz davon; und gegebenenfalls einen pharmazeutisch annehmbaren Träger.

11. Pharmazeutische Zusammensetzung nach Anspruch 9 oder Kombination nach Anspruch 10 zur Verwendung zur Behandlung oder Prävention von Dyslipidämie, Hyperlipidämie, Hypercholesterinämie, Atherosklerose, Hypertriglyceridämie, Herzversagen, Herzinfarkt, Gefäßerkrankungen, Herzkreislauferkrankungen, Bluthochdruck, Obesität, Entzündung, Arthritis, Krebs, Alzheimer-Erkrankung, Hautleiden, Atemwegserkrankungen, Augenerkrankungen, Entzündungserkrankungen des Darms, IBD (Reizdarmerkrankung), Colitis ulcerosa, Morbus Crohn, Erkrankungen, bei denen Glucosetoleranzstörungen, Hyperglykämie und Insulinresistenz eine Rolle spielen, wie z.B. Typ-1- und Typ-2-Diabetes, Störungen des Glucosestoffwechsels (IGM), Störungen der Glucosetoleranz (IGT), Fastenglucosestörungen (IFG) und Syndrom X.

12. Pharmazeutische Zusammensetzung nach Anspruch 9 oder Kombination nach Anspruch 10 zur Verwendung in einem Verfahren zur Behandlung oder Prävention des HIV-Wasting-Syndroms, einer schweren Langzeiterkrankung, eines Rückgangs der Muskelmasse und/oder der Muskelkraft, eines Rückgangs der Magermasse, zur Aufrechterhaltung von Muskelkraft und -funktion bei älteren Menschen sowie zur Behandlung und Prävention von reduzierter Muskelausdauer und Muskelfunktion sowie Gebrechlichkeit bei älteren Menschen.

13. Verbindung nach einem der Ansprüche 1 bis 5 oder pharmazeutische Zusammensetzung nach Anspruch 9 oder Kombination nach Anspruch 10 zur Verwendung in dem Behandlungsverfahren.

14. Verwendung einer Verbindung nach einem der Ansprüche 1 bis 5 oder einer pharmazeutischen Zusammensetzung nach Anspruch 9 oder einer Kombination nach Anspruch 10 zur Herstellung eines Medikaments zur Behandlung oder Prävention von Dyslipidämie, Hyperlipidämie, Hypercholesterinämie, Atherosklerose, Hypertriglyceridämie, Herzversagen, Herzinfarkt, Gefäßerkrankungen, Herzkreislauferkrankungen, Bluthochdruck, Obesität, Entzündung, Arthritis, Krebs, Alzheimer-Erkrankung, Hautleiden, Atemwegserkrankungen, Augenerkrankungen, Entzündungserkrankungen des Darms, IBD (Reizdarmerkrankung), Colitis ulcerosa, Morbus Crohn, Erkrankungen, bei denen Glucosetoleranzstörungen, Hyperglykämie und Insulinresistenz eine Rolle spielen, wie z.B. Typ-1- und Typ-2-Diabetes, Störungen des Glucosestoffwechsels (IGM), Störungen der Glucosetoleranz (IGT), Fastenglucosestörungen (IFG) und Syndrom X.

15. Verwendung einer Verbindung nach einem der Ansprüche 1 bis 5 oder einer pharmazeutischen Zusammensetzung nach Anspruch 9 oder einer Kombination nach Anspruch 10 zur Herstellung eines Medikaments zur Behandlung oder Prävention des HIV-Wasting-Syndroms, einer schweren Langzeiterkrankung, eines Rückgangs von Muskelmasse und/oder Muskelkraft, eines Rückgangs der Magermasse, zur Erhaltung von Muskelkraft und -funktion bei älteren Menschen, zur Behandlung oder Prävention von reduzierter Muskelausdauer und Muskelfunktion sowie Gebrechlichkeit bei älteren Menschen. ,

## Revendications

1. Composé de formule I: 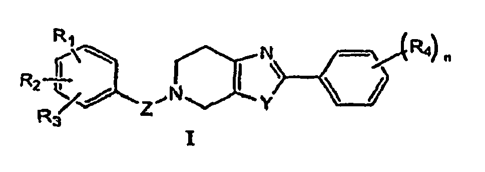 dans laquelle:
n est choisi parmi 0, 1 et 2;
R₁ est choisi parmi -XCO₂R₁₃ et -OCR₁₁R₁₂XCO₂R₁₃; où X est choisi parmi une liaison et un groupe alkylène en C_{1 à 4}; R₁₁ et R₁₂ sont indépendamment choisis parmi un atome d'hydrogène, un groupe alkyle en C_{1 à 4} et alcoxy en C_{1 à 4}; ou R₁₁ et R₁₂ conjointement avec l'atome de carbone auquel R₁₁ et R₁₂ sont attachés forment un groupe cycloalkyle en C_{3 à 12}; et R₁₃ est choisi parmi un atome d'hydrogène et un groupe alkyle en C_{1 à 6};
R₂ et R₃ sont indépendamment choisis parmi un atome d'hydrogène, un groupe halogéno, alkyle en C_{1 à 6}, alkyle en C_{1 à 6} substitué par halogéno, alcoxy en C_{1 à 6} et alcoxy en C_{1 à 6} substitué par halogéno;
Z est choisi parmi une liaison et -S(O)₀₋₂-;
Y est choisi parmi O et S;
R₄ est choisi parmi un atome d'hydrogène, un groupe halogéno, alkyle en C_{1 à 6}, alkyle en C_{1 à 6} substitué par halogéno, alcoxy en C_{1 à 6} et alcoxy en C_{1 à 6} substitué par halogéno, et sels, hydrates et solvates pharmaceutiquement acceptables de celui-ci.

2. Composé selon la revendication 1, dans lequel:
n est choisi parmi 0 et 1;
R₁ est choisi parmi -XCO₂R₁₃ et -OCR₁₁R₁₂XCO₂R₁₃; où X est choisi parmi une liaison et un groupe alkylène en C_{1 à 4}; R₁₁ et R₁₂ sont indépendamment choisis parmi un atome d'hydrogène, un groupe alkyle en C_{1 à 4} et alcoxy en C_{1 à 4}; ou R₁₁ et R₁₂ conjointement avec l'atome de carbone auquel R₁₁ et R₁₂ sont attachés forment un groupe cycloalkyle en C_{3 à 12}; et R₁₃ est choisi parmi un atome d'hydrogène et un groupe alkyle en C_{1 à 6};
R₂ et R₃ sont indépendamment choisis parmi un atome d'hydrogène, un groupe halogéno, alkyle en C_{1 à 6}, alkyle en C_{1 à 6} substitué par halogéno, alcoxy en C_{1 à 6} et alcoxy en C_{1 à 6}substitué par halogéno;
Z est choisi parmi une liaison et -S(O)₀₋₂-;
Y est choisi parmi O et S; et
R₄ est un groupe alkyle en C_{1 à 6} substitué par halogéno.

3. Composé selon la revendication 2, dans lequel: R₁ est choisi parmi -CH₂CO₂H, -(CH₂)₂CO₂H, -OC(CH₂)₂CO₂H et -OCH₂CO₂H.

4. Composé selon la revendication 3, dans lequel R₂ et R₃ sont indépendamment choisis parmi un atome d'hydrogène, un groupe méthyle et méthoxy; et R₄ est un groupe trifluorométhyle.

5. Composé selon la revendication 4, choisi parmi: l'acide {3-[2-(4-trifluorométhyl-phényl)-6,7-dihydro-4H-thiazolo[5,4-c]pyridin-5-yl]-phényl}-acétique; l'acide 3-{3-[2-(4-trifluorométhyl-phényl)-6,7-dihydro-4H-thiazolo[5,4-c]pyridin-5-yl]-phényl}-propionique; l'acide 3-{4-[2-(4-trifluorométhyl-phényl)-6,7-dihydro-4H-thiazolo[5,4-c]pyridin-5-yl]-phényl}-propionique; l'acide 2-méthyl-2-{2-méthyl-4-[2-(4-trifluorométhyl-phényl)-6,7-dihydro-4H-thiazolo[5,4-c]pyridin-5-yl]-phénoxy}-propionique; l'acide {4-méthyl-3-[2-(4-trifluorométhyl-phényl)-6,7-dihydro-4H-thiazolo[5,4-c]pyridine-5-sulfonyl]-phényl}-acétique; l'acide {4-méthoxy-3-[2-(4-trifluorométhyl-phényl)-6,7-dihydro-4H-thiazolo[5,4-c]pyridine-5-sulfonyl]-phényl}-acétique; l'acide {4-[2-(4-trifluorométhyl-phényl)-6,7-dihydro-4H-thiazolo[5,4-c]pyridine-5-sulfonyl]-phényl}-acétique; l'acide {2-méthyl-4-[2-(4-trifluorométhyl-phényl)-6,7-dihydro-4H-thiazolo[5,4-c]pyridine-5-sulfonyll-phénoxyl-acétique; l'acide 2-méthyl-2-{2-méthyl-4-[2-(4-trifluorométhyl-phényl)-6,7-dihydro-4H-thiazolo[5,4-c]pyridine-5-sulfonyl]-phénoxyl-propionique; et l'acide 2-{2,5-diméthyl-4-[2-(4-trifluorométhyl-phényl)-6,7-dihydro-4H-thiazolo[5,4-c]pyridine-5-sulfonyl]-phénoxy}-2-méthyl-propionique.

6. Utilisation d'un composé selon l'une quelconque des revendications 1 à 5 dans la fabrication d'un médicament destiné au traitement d'une maladie chez un animal où l'activité de PPAR contribue à la pathologie et/ou symptomatologie de la maladie.

7. Utilisation selon la revendication 6, dans laquelle l'activité de PPAR est au moins un PPAR choisi parmi le PPARα, le PPARδ et le PPARγ.

8. Utilisation selon la revendication 7, dans laquelle l'activité de PPAR est à la fois le PPARα et le PPARδ.

9. Composition pharmaceutique comprenant une quantité thérapeutiquement efficace d'un composé selon l'une quelconque des revendications 1 à 5 en combinaison avec un ou plusieurs excipients pharmaceutiquement acceptables.

10. Combinaison pharmaceutique, par exemple composition pharmaceutique, comprenant: 1) un composé selon l'une quelconque des revendications 1 à 5 ou un sel pharmaceutique acceptable de celui-ci; et 2) au moins un ingrédient actif choisi parmi:
a) les agents antidiabétiques tels que l'insuline, les dérivés d'insuline et les substances mimétiques; les sécrétagogues d'insuline tels que les sulfonylurées, par exemple, le glipizide, le glyburide et l'amaryl; les ligands du récepteur de sulfonylurées insulinotropes tels que les méglitinides, par exemple, le natéglinide et le répaglinide; un médicament insulinosensibilisant tel que les inhibiteurs de protéine tyrosine phosphatase-1B (PTP-1B) tels que PTP-112; les inhibiteurs de GSK3 (glycogène synthase kinase-3) tels que SB-517955, SB-4195052, SB-216763, NN-57-05441 et NN-57-05445; les ligands de RXR tels que GW-0791 et AGN-194204; les inhibiteurs cotransporteurs de glucose dépendant du sodium tels que T-1095; les inhibiteurs de glycogène phosphorylase A tels que BAY R3401; les biguanides tels que la metformine; les inhibiteurs d'alpha-glucosidase tels que l'acarbose; GLP-1 (peptide de type glucagon-1), les analogues de GLP-1 tels que l'exendine-4 et les substances mimétiques de GLP-1; les inhibiteurs de dipeptidyl peptidase IV tels que DPP728, la vildagliptine, MK-0431, la saxagliptine, GSK23A; un casseur AGE; un dérivé de thiazolidone (glitazone) tel que la pioglitazone, la rosiglitazone, ou l'acide (R)-1-{4-[5-méthyl-2-(4-trifluorométhyl-phényl)-oxazol-4-ylméthoxy]-benzènesulfonyl}-2,3-dihydro-1H-indole-2-carboxylique, un agoniste de PPARγ de type non-glitazone, par exemple GI-262570;
b) les agents hypolipidémiques tels que les inhibiteurs de 3-hydroxy-3-méthyl-glutaryl coenzyme A (HMG-CoA) réductase, par exemple, la lovastatine, la pitavastatine, la simvastatine, la pravastatine, la cérivastatine, la mévastatine, la vélostatine, la fluvastatine, la dalvastatine, l'atorvastatine, la rosuvastatine et la rivastatine; les inhibiteurs de squalène synthase; les ligands de FXR (récepteur X de farnésoïde) et LXR (récepteur X du foie); la cholestyramine; les fibrates; l'acide nicotinique et l'aspirine;
c) un agent anti-obésité ou un agent régulateur de l'appétit tel que la phentermine, la leptine, la bromocriptine, la dexamphétamine, l'amphétamine, la fenfluramine, la dexfenfluramine, la sibutramine, l'orlistat, la dexfenfluramine, le mazindol, la phentermine, la phendimétrazine, le diéthylpropion, la fluoxétine, le bupropion, le topiramate, le diéthylpropion, la benzphétamine, la phénylpropanolamine ou l'écopipam, l'éphédrine, la pseudoéphédrine ou les antagonistes du récepteur cannabinoïde;
d) les agents anti-hypertenseurs; par exemple, les diurétiques de l'anse de Henle tels que l'acide éthacrynique, le furosémide et le torsémide; les diurétiques tels que les dérivés de thiazide, le chlorithiazide, l'hydrochlorothiazide, l'amiloride; les inhibiteurs de l'enzyme de conversion de l'angiotensine (ACE) tels que le bénazépril, le captopril, l'énalapril, le fosinopril, le lisinopril, le moexipril, le périnodopril, le quinapril, le ramipril et le trandolapril; les inhibiteurs de la pompe à membrane Na-K-ATPase tels que la digoxine; les inhibiteurs de neutralendopeptidase (NEP) tels que le thiorphan, le tertéo-thiorphan, SQ29072; les inhibiteurs d'ECE, par exemple SLV306; les inhibiteurs d'ACE/NEP tels que l'omapatrilat, le sampatrilat et le fasidotril; les antagonistes de l'angiotensine II tels que le candésartan, l'éprosartan, l'irbésartan, le losartan, le telmisartan et le valsartan, en particulier le valsartan; les inhibiteurs de rénine tels que l'aliskirène, le terlakirène, le ditékirène, R066-1132, RO-66-1168; les bloqueurs du récepteur β-adrénergique tels que l'acébutolol, l'aténolol, le bétaxolol, le bisoprolol, le métoprolol, le nadolol, le propranolol, le sotalol et le timolol; les agents inotropes tels que la digoxine, la dobutamine et la milrinone; les bloqueurs des canaux calciques tels que l'amlodipine, le bépridil, le diltiazem, la félodipine, la nicardipine, la nimodipine, la nifédipine, la nisoldipine et le vérapamil; les antagonistes du récepteur d'aldostérone; et les inhibiteurs de l'aldostérone synthase;
e) un composé d'augmentation de HDL;
f) un modulateur de l'absorption de cholestérol tel que Zetia® et KT6-971;
g) les analogues et substances mimétiques Apo-A1;
h) les inhibiteurs de thrombine tels que le ximélagatran;
i) les inhibiteurs d'aldostérone tels que l'anastrazole, le fadrazole, l'éplérénone;
j) les inhibiteurs d'agrégation plaquettaire tels que l'aspirine, le bisulfate de clopidogrel;
k) l'oestrogène, la testostérone, un modulateur du récepteur d'oestrogène sélectif, un modulateur du récepteur d'androgène sélectif;
l) un agent chimiothérapeutique tel que les inhibiteurs d'aromatase par exemple le femara, les anti-oestrogènes, les inhibiteurs de topoisomérase I, les inhibiteurs de topoisomérase II, les agents actifs microtubulaires, les agents d'alkylation, les antimétabolites antinéoplasiques, les composés platine, les composés diminuant l'activité de la protéine kinase tels qu'un inhibiteur de tyrosine kinase du récepteur de PDGF de préférence l'imatinib ou le 4-méthyl-N-[3-(4-méthyl-imidazol-1-yl)-5-trifluorométhyl-phényl]-3-(4-pyridin-3-yl-pyrimidin-2-ylamino)-benzamide, et
m) un agent interagissant avec un récepteur de 5-HT₃ et/ou un agent interagissant avec un récepteur de 5-HT₄ tel que le tégasérod, l'hydrogénomaléate de tégasérod, le cisapride, le cilansétron;
ou, dans chaque cas, un sel pharmaceutiquement acceptable de celui-ci; et facultativement un support pharmaceutiquement acceptable.

11. Composition pharmaceutique selon la revendication 9, ou combinaison selon la revendication 10, pour une utilisation dans un procédé de traitement ou de prévention de la dyslipidémie, de l'hyperlipidémie, de l'hypercholestérolémie, de l'athérosclérose, de l'hypertriglycéridémie, d'une attaque cardiaque, d'un infarctus du myocarde, de maladies vasculaires, de maladies cardiovasculaires, de l'hypertension, de l'obésité, d'une inflammation, de l'arthrite, d'un cancer, de la maladie d'Alzheimer, des affections cutanées, des troubles respiratoires, des troubles ophtalmiques, des affections abdominales inflammatoires, de l'IBD (syndrome du côlon irritable), de la colite ulcéreuse, de la maladie de Crohn, des affections dans lesquelles l'intolérance au glucose, l'hyperglycémie et la résistance à l'insuline sont impliquées, telles que le diabète de type I et de type II, la défaillance du métabolisme glucosique (IGM), l'intolérance au glucose (IGT), l'hyperglycémie modérée à jeun (IFG) et le syndrome X.

12. Composition pharmaceutique selon la revendication 9 ou combinaison selon la revendication 10, pour une utilisation dans un procédé de traitement ou de prévention du syndrome de dépérissement du VIH, d'une maladie grave à long terme, d'une diminution de la masse musculaire et/ou d'une diminution de la tonicité musculaire, d'une diminution de la masse maigre de l'organisme, d'un maintien de la tonicité musculaire et de la fonction musculaire chez les personnes âgées, d'une diminution de l'endurance musculaire et de la fonction musculaire, et d'une fragilité chez les personnes âgées.

13. Composé selon l'une quelconque des revendications 1 à 5, ou composition pharmaceutique selon la revendication 9 ou combinaison selon la revendication 10, pour une utilisation dans un procédé de traitement.

14. Utilisation d'un composé selon l'une quelconque des revendications 1 à 5, ou d'une composition pharmaceutique selon la revendication 9 ou d'une combinaison selon la revendication 10, pour la fabrication d'un médicament destiné au traitement ou à la prévention de la dyslipidémie, de l'hyperlipidémie, de l'hypercholestérolémie, de l'athérosclérose, de l'hypertriglycéridémie, d'une attaque cardiaque, d'un infarctus du myocarde, de maladies vasculaires, de maladies cardiovasculaires, de l'hypertension, de l'obésité, d'une inflammation, de l'arthrite, d'un cancer, de la maladie d'Alzheimer, des affections cutanées, des troubles respiratoires, des troubles ophtalmiques, des affections abdominales inflammatoires, de l'IBD (syndrome du côlon irritable), de la colite ulcéreuse, de la maladie de Crohn, des affections dans lesquelles l'intolérance au glucose, l'hyperglycémie et la résistance à l'insuline sont impliquées, telles que le diabète de type I et de type II, la défaillance du métabolisme glucosique (IGM), l'intolérance au glucose (IGT), l'hyperglycémie modérée à jeun (IFG) et le syndrome X.

15. Utilisation d'un composé selon l'une quelconque des revendications 1 à 5, ou d'une composition pharmaceutique selon la revendication 9 ou d'une combinaison selon la revendication 10, pour la fabrication d'un médicament destiné au traitement ou à la prévention du syndrome de dépérissement du VIH, d'une maladie grave à long terme, d'une diminution de la masse musculaire et/ou de la tonicité musculaire, d'une diminution de la masse maigre de l'organisme, d'un maintien de la tonicité musculaire et de la fonction musculaire chez les personnes âgées, d'une diminution de l'endurance musculaire et de la fonction musculaire, et d'une fragilité chez les personnes âgées.
